(19) Europäisches Patentamt European Patent Office Office européen des brevets

(11) **EP 4 070 670 A1**

(12) **EUROPEAN PATENT APPLICATION**

(43) Date of publication:
**12.10.2022 Bulletin 2022/41**

(21) Application number: **21167470.0**

(22) Date of filing: **08.04.2021**

(51) International Patent Classification (IPC):
*A23L 33/135* (2016.01)    *A23L 33/00* (2016.01)
*A61K 35/747* (2015.01)    *A61P 1/00* (2006.01)
*A61P 3/10* (2006.01)    *A61P 37/08* (2006.01)
*C12N 1/20* (2006.01)    *C12R 1/225* (2006.01)
*A61P 37/02* (2006.01)

(52) Cooperative Patent Classification (CPC):
**A23L 33/135; A61K 35/747; A61P 1/00;**
**A61P 3/10; A61P 37/02; A61P 37/08; C12N 1/205;**
A23Y 2220/63; C12N 9/2402; C12R 2001/225;
C12Y 302/01065

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **University College Cork-National University of Ireland Cork**
**Cork (IE)**
• **Agriculture and Food Development Authority (TEAGASC)**
**Co. Carlow (IE)**

(72) Inventors:
• **O'Toole, Paul**
**Co. Cork (IE)**
• **van Sinderen, Douwe**
**Co. Cork (IE)**
• **Ross, Paul**
**Co. Cork (IE)**
• **Melgar, Silvia**
**Co. Cork (IE)**

(74) Representative: **Purdylucey Intellectual Property**
**6-7 Harcourt Terrace**
**D02 FH73 Dublin 2 (IE)**

(54) **LACTICASEIBACILLUS PARACASEI EM025-11 AND USES THEREOF**

(57)    A strain of *Lacticaseibacillus paracasei* EM205-11 is described. The strain has an unusually broad catabolic activity including for prebiotics not normally utilized by commensal lactobacilli (Fig. 13) and is therefore suitable for inclusion in a synbiotic combination. The strain has also been found to adhere to but did not invade intestinal epithelial cells (IECs), which is an important probiotic trait (Fig. 1), and also demonstrates anti-inflammatory activity.

FIGURE 13A

**(Cont. next page)**

EP 4 070 670 A1

**Therapeutic Tx , 7d- Proximal Colon**

FIGURE 13B

**Therapeutic Tx , 7d - Distal Colon**

FIGURE 13C

**Therapeutic Tx, 7d - *In vivo* Permeability**

FIGURE 13D

**Description**

Field of the Invention

The present invention relates to an isolated strain of *Lacticaseibacillus paracasei,* compositions comprising the strain, and uses of the strain or composition.

Background to the Invention

*L. casei* strains are popular in the marketplace for relieving bloating, a feature of IBS and more general intestinal discomfort. Prebiotics are indicated for IBS-C, the constipation subtype of IBS. They are also used to promote SCFA production, and as such suitable for the elderly (e.g. reverse frailty by supply of energy to colonocytes). SCFAs promote intestinal barrier function and may be indicated for conditions where leaky gut is implicated, such as IBD. Lactobacilli are desirable in probiotic products, but traditionally they are not suited for synbiotic products because they normally lack the enzymatic 'machinery' to break down complex carbohydrates.

It is an object of the invention to overcome at least one of the above-referenced problems.

Summary of the Invention

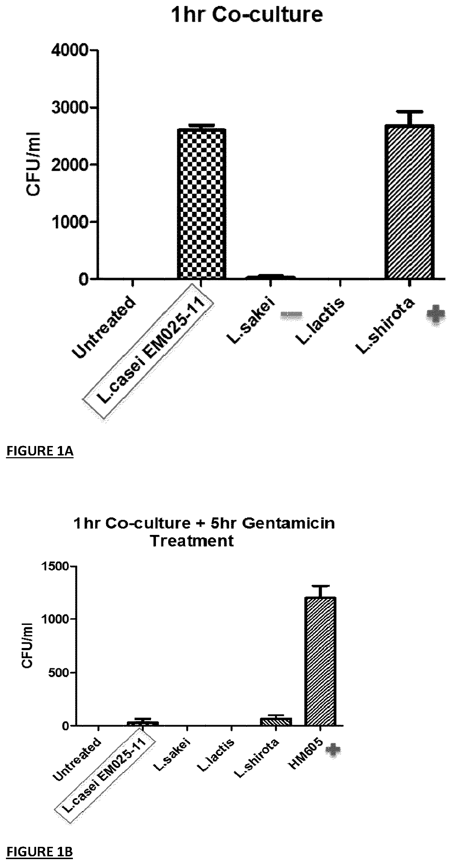

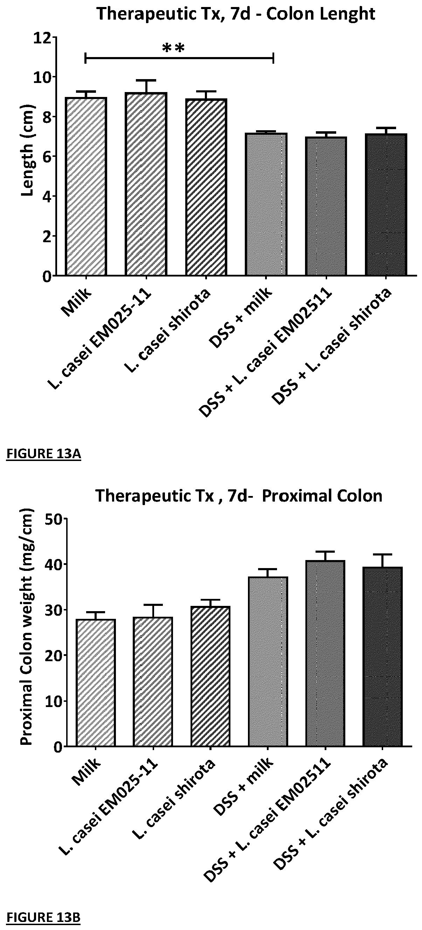

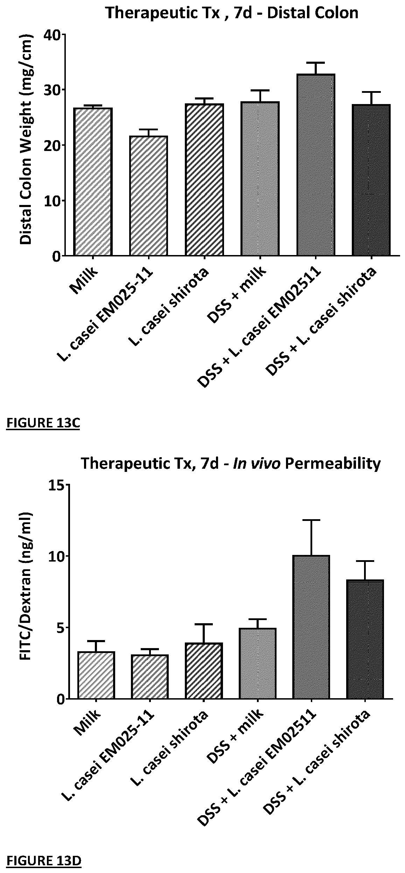

The Applicant has isolated and extensively characterized a bacterium, *Lacticaseibacillus paracasei,* (*L. paracasei*) EM025-11, that exhibits a number of important probiotic strains. The strain has an unusually broad catabolic activity including for prebiotics not normally utilized by commensal lactobacilli (Fig. 13) and is therefore suitable for inclusion in a synbiotic combination. The strain has also been found to adhere to but did not invade intestinal epithelial cells (IECs), which is an important probiotic trait (Fig. 1A-B). The strain also demonstrates anti-inflammatory activity - it has an anti-inflammatory profile in IECs, upregulating genes associated with immune engagement in IECs, is immunomodulatory in a PBMC assay and also shows immunomodulatory activity in in-vitro activated murine splenocytes and mesenteric lymph node cells (MLNs). The strain also improves gut barrier function without the bacterium crossing the barrier or provoking cell death. The Applicant also shows that *L. paracasei* EM025-11 improves gut barrier function by upregulating genes associated with gut barrier function.

Moreover, *in vivo* results with the strain also showed probiotic potential. Daily oral gavage with *L paracasei* EM025-11 in milk resulted in higher faecal bacteria recovery. Furthermore, therapeutic treatment with *L. paracasei* EM025-11 for 14 days had the following effects:

-    improved clinical signs of colitis
-    improved colon macroscopic and histology scores
-    reduced plasma and colon pro-inflammatory cytokines and STAT3 mRNA expression in colon without the induction of anti-inflammatory mediators
-    reduced colon E-cadherin and Muc2 mRNA expression without an effect on expression of genes for tight junction proteins or anti-microbial peptides

According to a first aspect of the present invention, there is provided an isolated *L. paracasei* EM205-11 strain as deposited with the National Collection of Industrial and Marine Bacteria under the Accession No. NCIMB 43703 on 17 December 2020 (hereafter "EM205-11 strain" or "strain of the invention" or "deposited strain").

The invention also relates to mutants and variants of the deposited strain that are substantially identical, genetically and phenotypically, to the deposited strain and retain the most or all of the activity of the deposited strain. Thus, the invention relates to derivatives of the strain that have been genetically engineered to modify the genome of the strain, typically without fundamentally altering the functionality of the organism, for example engineered for heterologous expression of a nucleic acid, or engineered to overexpress an endogenous gene, or engineered to silence a gene, to produce a recombinant or transformed strain of the invention. Genetic modifications may be carried out using recombinant DNA techniques and reagents, including gene editing technologies such as CRISP-Cas9 techniques (see below). The term also includes variants of the strain having natural or spontaneous genetic alterations. The term is also intended to encompass variant strains obtained by serial passage of the isolated strain of the invention.

The invention also relates to a supernatant or cell material derived from the isolated EM205-11 strain or mutant or variant thereof.

The invention also provides a composition comprising the isolated EM205-11 strain, or a supernatant or cell material derived from the isolated EM205-11 strain, or mutant or variant thereof.

The composition may be a synbiotic composition (typically a synergistic synbiotic composition) comprising the isolated EM205-11 strain, or a supernatant or cell material derived from the isolated EM205-11 strain, or mutant or variant thereof, in combination with a prebiotic material, for example a long chain carbohydrate. The prebiotic may comprise dietary fibre, especially soluble dietary fibre. The prebiotic material may comprise an inulin carbohydrate.

The invention also provides a composition comprising an isolated *L. paracasei* strain, in combination with a prebiotic material, for example a long chain carbohydrate. In one embodiment, the isolated *L. paracasei* strain is a probiotic strain. In one embodiment, the isolated *L. paracasei* strain expresses a levanase. In one embodiment, the isolated *L. paracasei* strain expresses a levanase having an amino acid sequence that is at least 70%, 80%, 90%, 91%, 92%, 93%, 94%, 95%, 96%, 97%, 98%, 99%, 99.1%, 99.2%, 99.3%, 99.4%, 99.5%, 99.6%, 99.7%, 99.8% or 99.9% identical with SEQUENCE ID NO:1. In one embodiment, the isolated *Lactobacillus casei* strain is derived from the strain of the invention.

The composition may be a pharmaceutical composition and may include a suitable pharmaceutical excipient. The composition may be provided in a unit dose form suitable for oral administration, e.g. a tablet or capsule.

The composition may be a food or beverage product, or a nutritional supplement. The composition may be an infant formula. The composition may be a complete food for an elderly or immunocompromised subject.

The composition may comprise a probiotic material. The composition may comprise a prebiotic material.

The composition may comprise an additional probiotic bacterium.

The strain in the composition may be viable or non-viable, and may comprise a strain extract (e.g. bacterial cell lysate) or supernatant derived from the strain. The extract or supernatant may be in any physical form, for example liquid or dried.

The composition may comprise at least $10^6$ cfu per gram of the strain.

The composition may be solid or liquid. The composition may comprise a carrier for oral delivery. The carrier may be in the form of tablet, capsule, powder, granules, microparticles or nanoparticles. The carrier may be configured for targeted release in the intestine (i.e. configured for gastric transit and ileal release). The carrier may be configured for controlled release in the intestine (e.g. configured for gastric transit and ileal release).

The composition may be dried or lyophilised.

The invention also relates to a method of treating or preventing a disease or condition in a subject by administering to the subject:

(a) the isolated EM205-11 strain, or a supernatant or cell material derived from the isolated EM205-11 strain, or mutant or variant thereof; or

(b) a composition comprising (a),

in which the disease or condition is selected from:

Intestinal discomfort;
Bloating;
Constipation;
Leaky gut syndrome;
Irritable bowel syndrome (or a symptom thereof);
Inflammatory bowel disease (or a symptom thereof);
Ulcerative colitis;
Crohn's disease;
Metabolic syndrome; and
An allergic disease.

In any embodiment, the method comprises administering a synbiotic composition to the subject.

The invention also provides a method of improving or maintaining gut health in a subject comprising a step of administering to the subject:

(a) the isolated EM205-11 strain, or a supernatant or cell material derived from the isolated EM205-11 strain, or mutant or variant thereof; or

(b) a composition comprising (a).

The invention also provides a method of treating or preventing a disease or condition characterised by a leaky gut in a subject comprising a step of administering to the subject:

(a) the isolated EM205-11 strain, or a supernatant or cell material derived from the isolated EM205-11 strain, or mutant or variant thereof; or

(b) a composition comprising (a).

The invention also provides a method of improving or promoting intestinal barrier function in a subject comprising a step of administering to the subject:

(a) the isolated EM205-11 strain, or a supernatant or cell material derived from the isolated EM205-11 strain, or mutant or variant thereof.; or

(b) a composition comprising (a).

The invention also provides a method of treating or preventing inflammation or an inflammatory disorder in a subject comprising a step of administering to the subject:

(a) the isolated EM205-11 strain, or a supernatant or cell material derived from the isolated EM205-11 strain, or mutant or variant thereof; or

(b) a composition comprising (a).

In one embodiment, the inflammatory disorder is a gut inflammatory disorder for example Inflammatory Bowel Disease or a similar condition.

In one embodiment, the inflammation is gut inflammation associated with an inflammatory disorder of the gut, for example Inflammatory Bowel Disease or a similar condition.

The invention also provides a method of producing a supernatant from an isolated *L. paracasei* EM205-11 strain comprising a step of culturing the isolated strain and separating the supernatant from the strain.

The invention also provides a method of producing an extract from an isolate comprising *L. paracasei* EM205-11 strain a step of lysing the cell and separating the cell extract from lysed cell material.

The invention also provides a supernatant or bacterial material or extract (for example a cell lysate) formed according to the method of the invention.

The invention also relates to a method of producing a derivative (e.g. mutant or variant) of the strain of the invention that have been genetically engineered to modify the genome of the strain, typically without fundamentally altering the functionality of the organism, for example engineered for heterologous expression of a nucleic acid, or engineered to overexpress an endogenous gene, or engineered to silence a gene, to produce a recombinant or transformed strain of the invention. Typically, the derivative is culturable. Typically, the derivative expresses a functional levanase protein.

In one embodiment, the subject is overweight (BMI > 25). In one embodiment, the subject is obese (BMI > 30). In one embodiment, the subject's weight ranges between a BMI of 20 to 25.

In one embodiment, the subject is an adult. In one embodiment, the subject is an infant. In one embodiment, the subject is an child. In one embodiment, the subject is an elderly subject (>70 years).

In one embodiment, the composition is selected from a pharmaceutical composition, a food, a food supplement, or a beverage.

The invention also provides an isolated EM205-11 strain for use as a medicament.

The invention also provides a cell extract or supernatant of an isolated EM205-11 strain, for use as a medicament.

Other aspects and preferred embodiments of the invention are defined and described in the other claims set out below.

Brief Description of the Figures

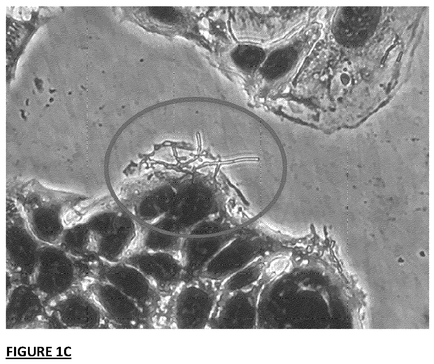

Figure 1: *L. paracasei* EM025-11 adheres (A) to but does not invade (B) intestinal epithelial cells (IECs). Positive control = adherent and invasive *E. coli* strain (AIEC)-HM605. n=3

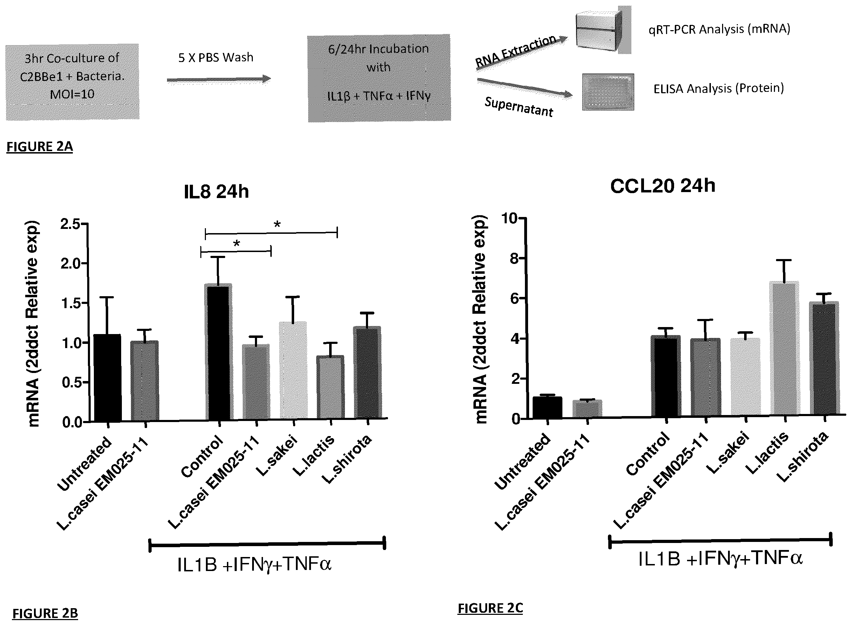

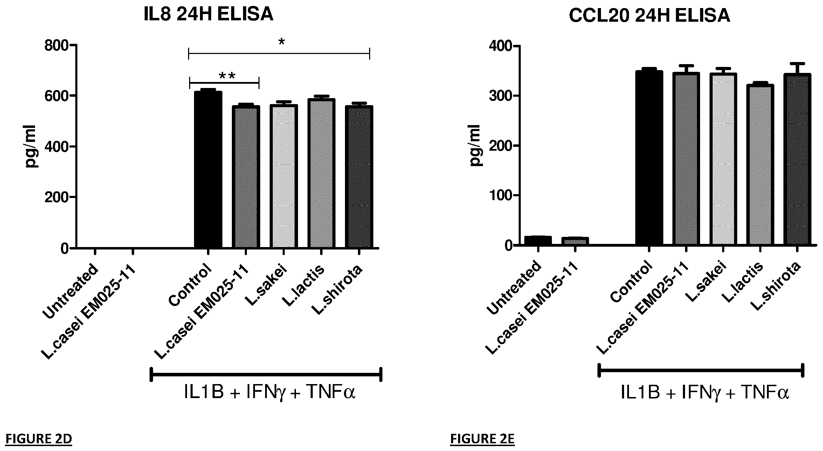

Figure 2: (A) Schematic of the co-culture of *Lactobacillus* sp. strains with human intestinal epithelial C2BBe1 cells. *L. paracasei* EM025-11 reduces the mRNA expression of the pro-inflammatory chemokines (B) IL-8 and (C) CCL20 induced by the pro-inflammatory cytokine cocktail containing IL-1$\beta$, IFN$\gamma$ and TNF$\alpha$. Two tailed t-test, *p<0.05 compared to control. n=3.

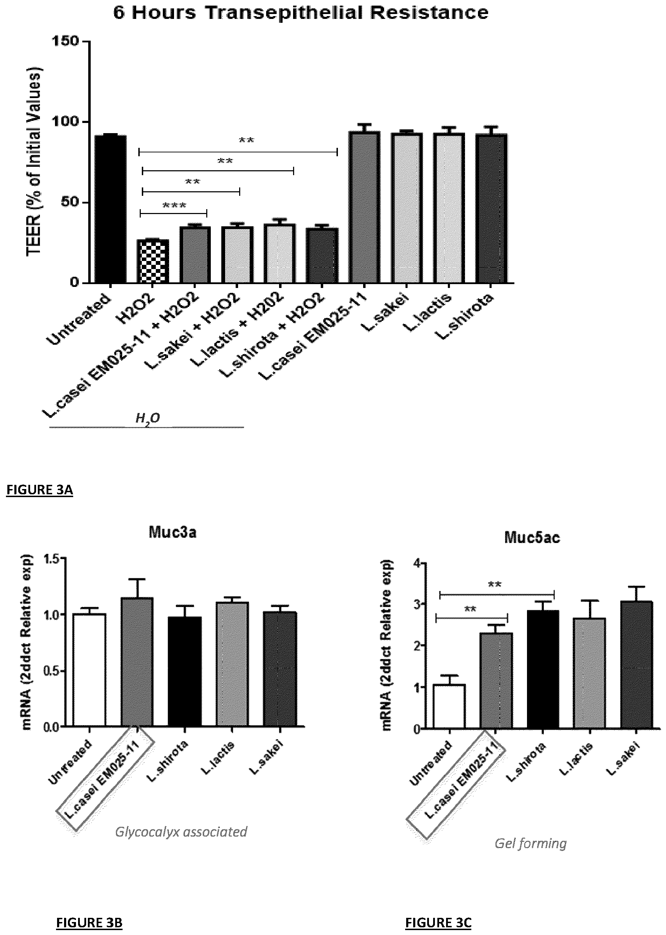

Figure 3: *L. paracasei* EM025-11 improves (A) barrier function [transepithelial resistance, TEER] and increases the mRNA expression of the barrier function-related genes (B) Muc3a and (C) Muc5a. Two tailed t-test, **p<0.01; ***p<0.001 against untreated cells. n=3-4.

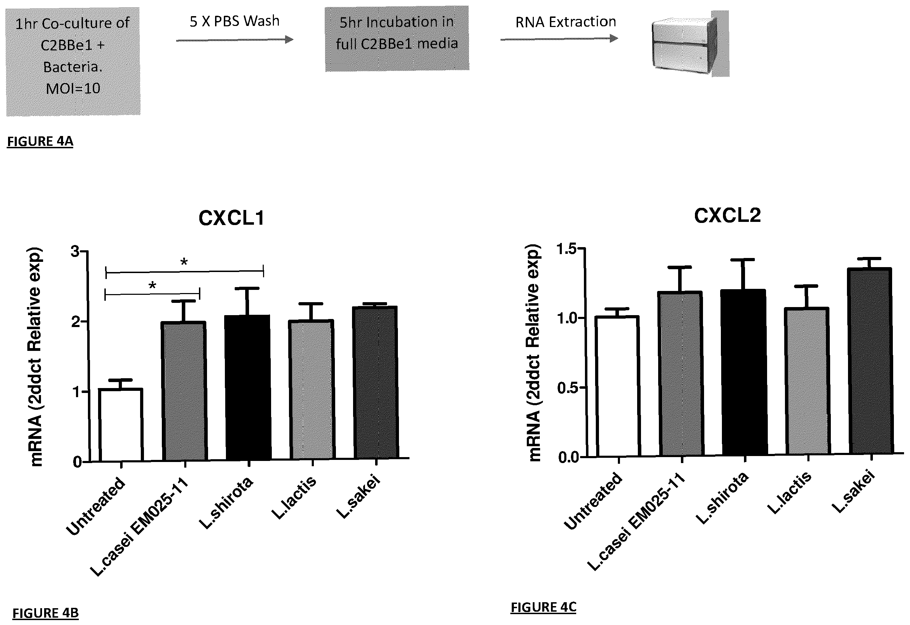

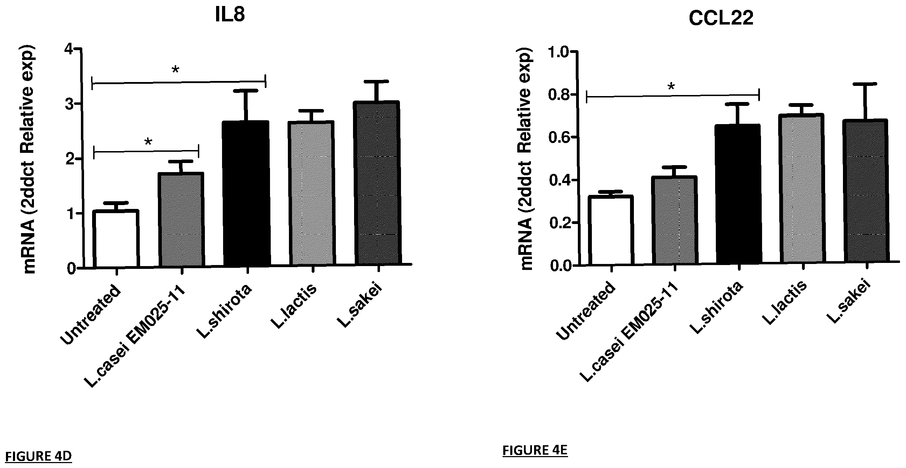

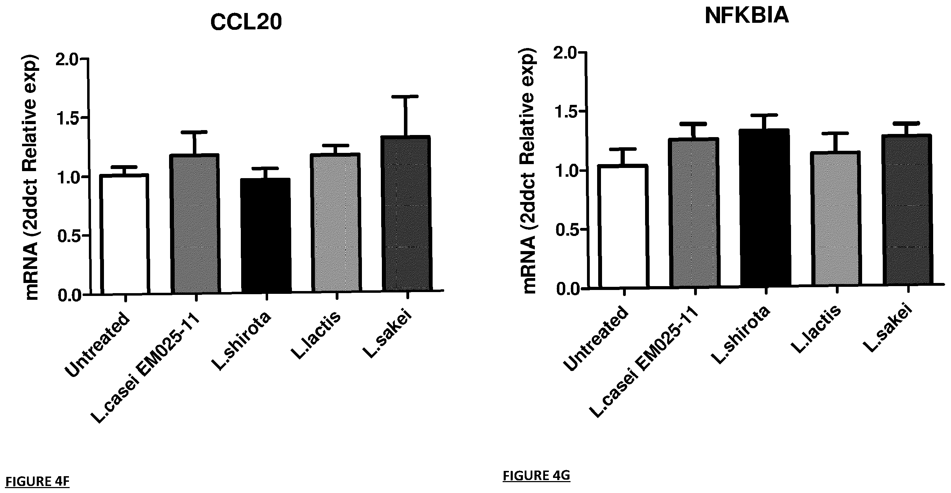

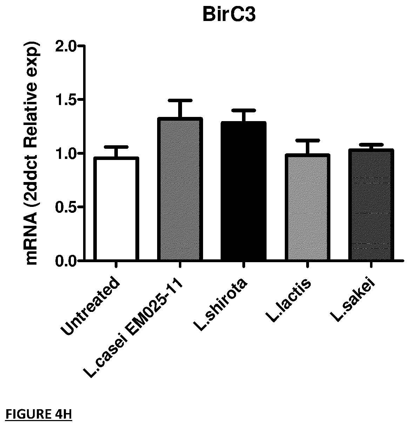

**Figure 4:** (A) Schematic of the co-culture of *Lactobacillus* sp. strains in Peripheral blood mononuclear (PBMC) assay. *L. paracasei* EM025-11 promotes the secretion of the innate cytokines (B) IL-6 and (C) IL-8; T helper 1 cytokines (D) IL-12p70 and (E) IFNy and T helper 2 cytokines (F) IL-4 and (G) IL-10 as measured by Meso Scale Discovery (MSD) technology. Positive control - TLR1/2 Agonist Pam3CSK4. Two tailed t-test, *p<0.05; **p<0.01; ***p<0.001 compared to untreated. n=2.

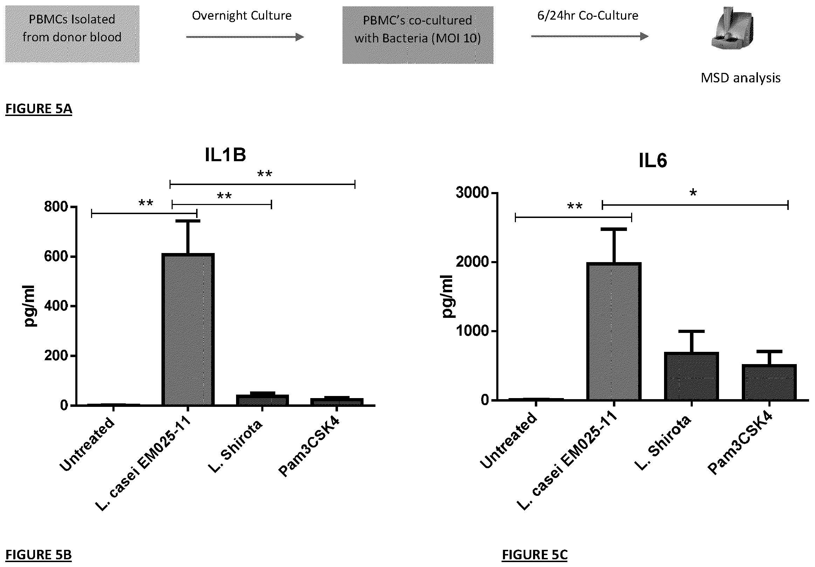

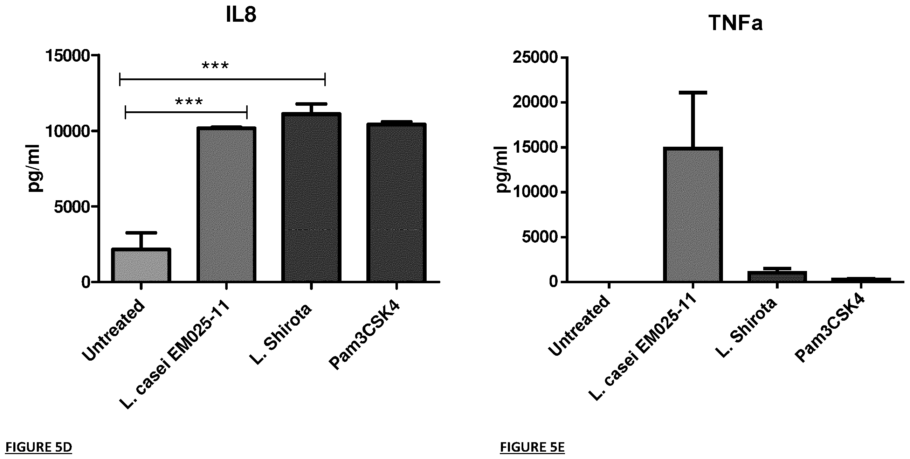

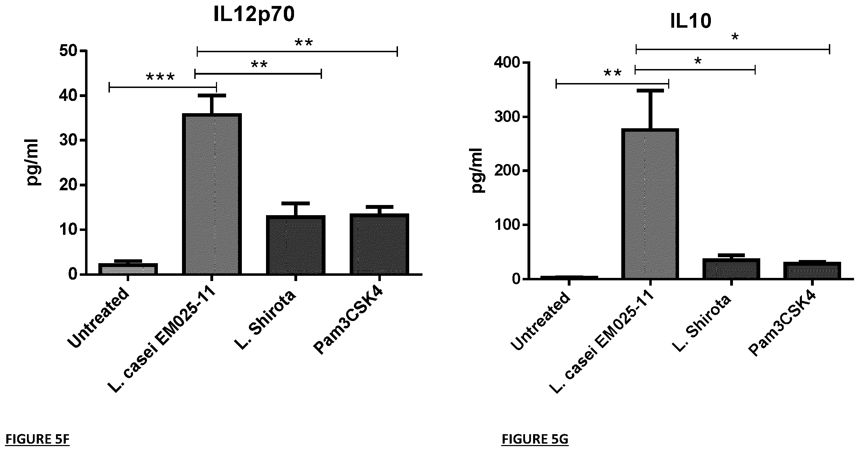

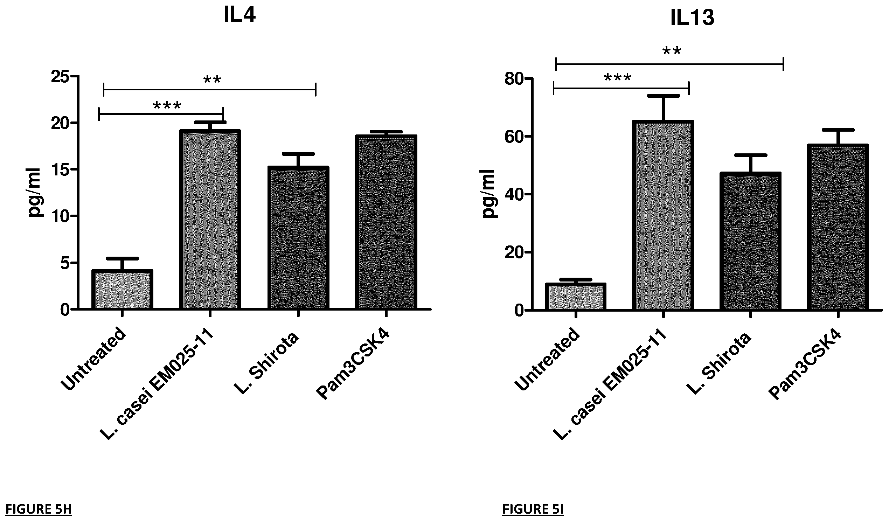

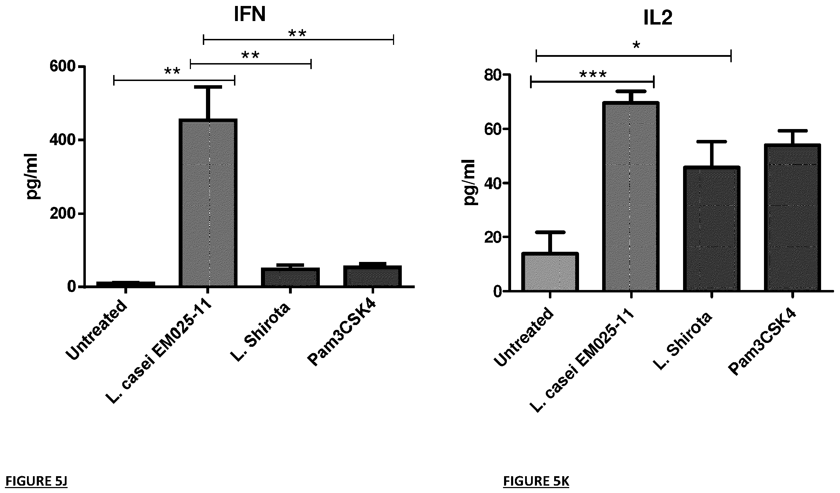

**Figure 5:** (A) Schematic of the oral gavage of *Lactobacillus* sp. strains to BALB/c mice. (B) Higher faecal bacterial count yield in BALB/c mice orally gavaged with milk-based *Lactobacillus* solution. Control groups - PBS and 2% Non-fat milk. n=10 mice/ group.

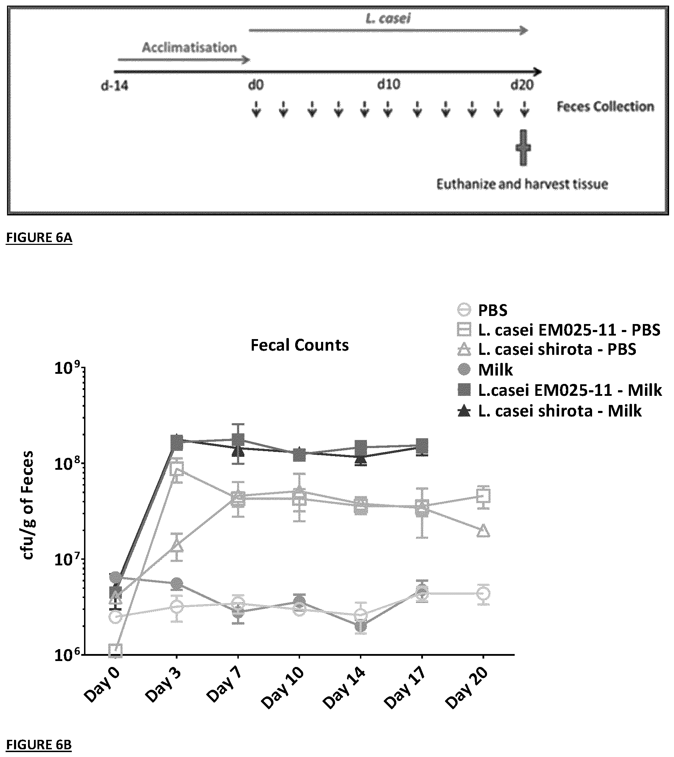

**Figure 6**: (A) Schematic of the isolation and culture of splenocytes from BALB/c mice orally gavaged for 20 days with *Lactobacillus* sp. strains. Splenocytes from *L. paracasei* EM025-11 gavaged mice present an altered secretion of pro-inflammatory (B) IL-6 and (C) mKC (murine IL-8) and anti-inflammatory (C) IL-10 upon culture in either culture media or with lipopolysaccharide (LPS) and Phorbol 12-myristate 13-acetate (PMA)/Ionomycin (Iono), measured by MSD technology. Two tailed t-test, *p<0.05; **p<0.01; ***p<0.001 compared to untreated cells. n=3 per treatment per group.

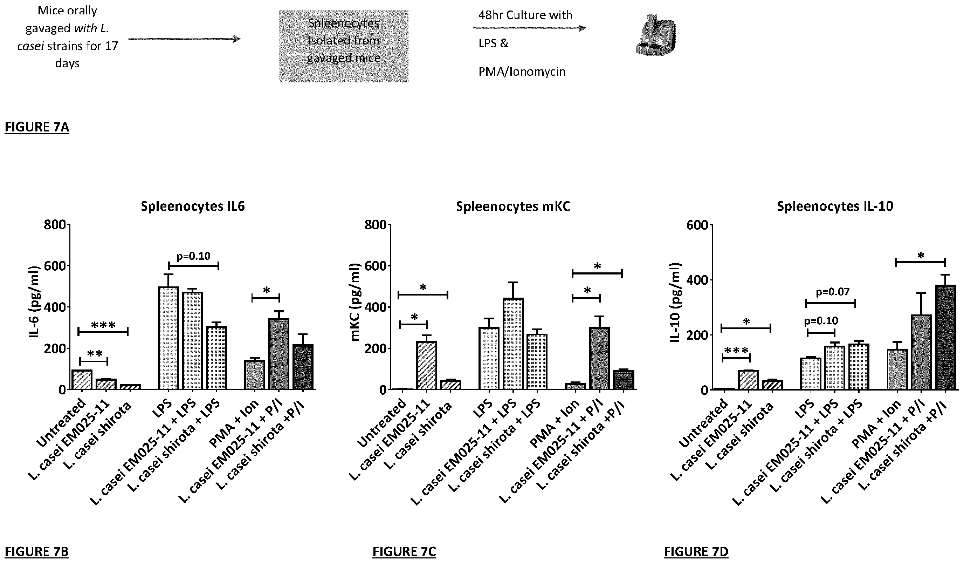

**Figure 7**: (A) Schematic of the isolation and culture of mesenteric lymph node (MLN) cells from BALB/c mice orally gavaged for 20 days with *Lactobacillus* sp. strains. MLNs from *L. paracasei* EM025-11 gavaged mice present an altered secretion of pro-inflammatory (B) IL-6 and (C) mKC (murine IL-8) and an increased secretion of anti-inflammatory (C) IL-10 upon culture in either culture media or with lipopolysaccharide (LPS) and Phorbol 12-myristate 13-acetate (PMA)/Ionomycin (Iono), measured by MSD technology. Two tailed t-test, *p<0.05; **p<0.01 compared to untreated cells. n=3 per treatment per group.

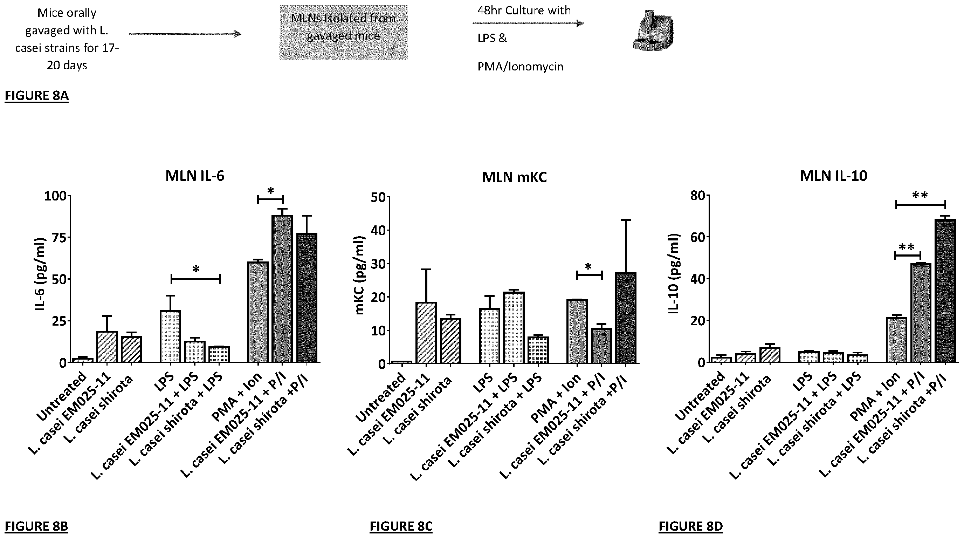

**Figure 8**: (A) Schematic of the oral gavage with *Lactobacillus* sp. strains for 14 days to BALB/c mice with DSS-induced colitis. *L. paracasei* EM025-11 improves (B) body weight loss, (C) colon weight and (D) histology. Control group - 2% Non-fat milk. One-way ANOVA, post-hoc, *p<0.05; **p<0.01. n=9-10 mice/DSS groups, n=5 mice/Control groups. For histology analysis n=5-7 mice/group.

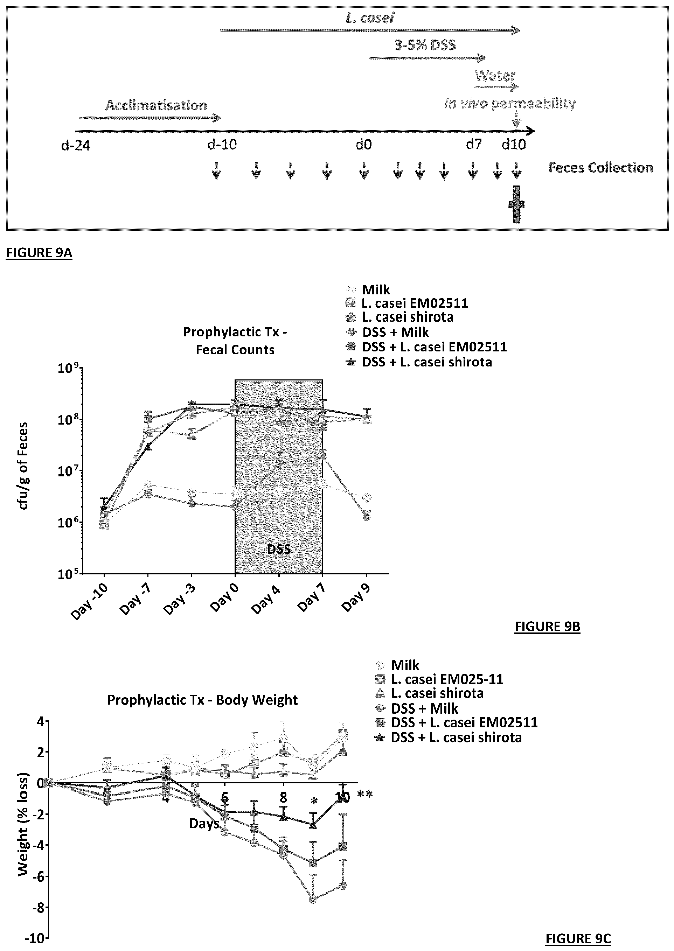

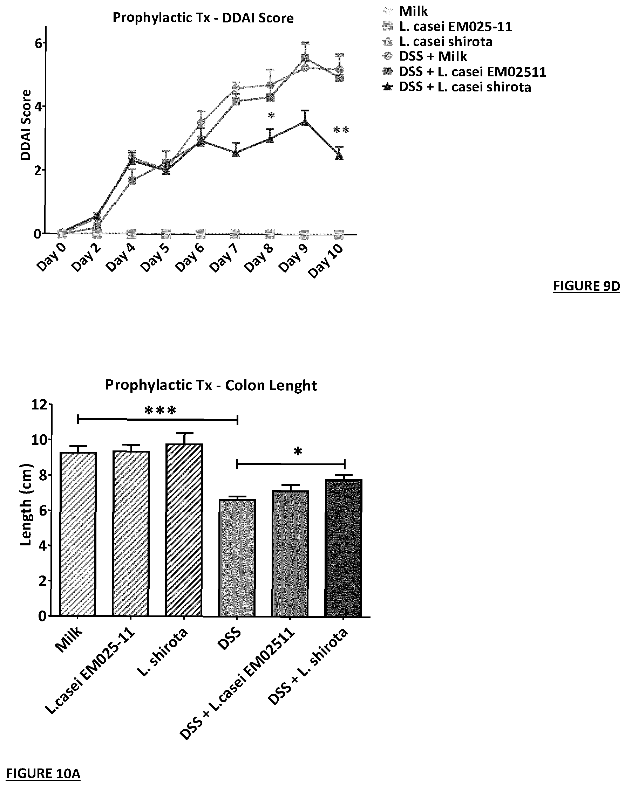

**Figure 9:** Plasma levels of (A) IL-6 and (B) mKC are reduced by *L. paracasei* EM025-11 gavage of BALB/c mice with DSS-induced colitis. Control group - 2% Non-fat milk. n=9-10 mice/DSS groups, n=5 mice/Control groups.

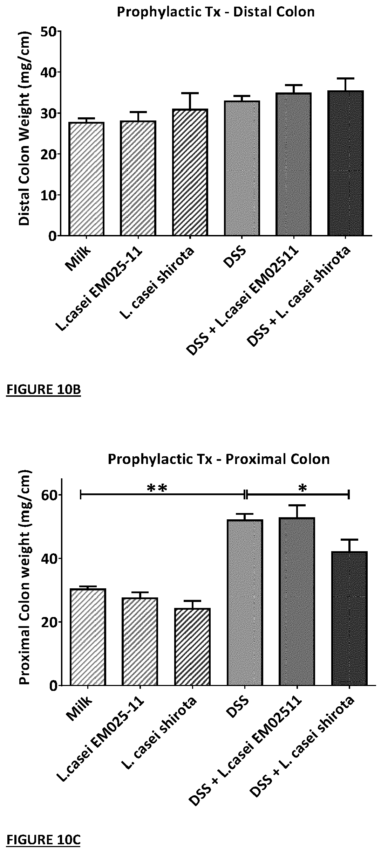

**Figure 10:** Colon levels of pro-inflammatory (A) mKC and (B) IL-6 and anti-inflammatory (C) IL-10 are reduced by *L. paracasei* EM025-11 gavage of BALB/c mice with DSS-induced colitis. Control group - 2% Non-fat milk. n=9-10 mice/DSS groups, n=5 mice/Control groups.

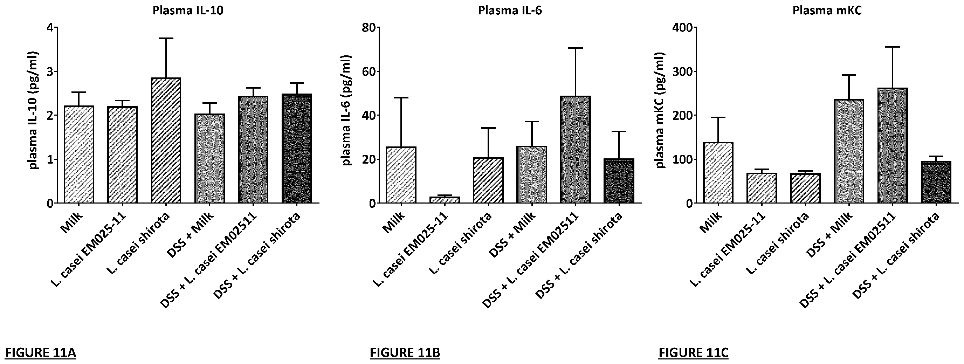

**Figure 11:** Colon mRNA expression of the inflammatory markers (A) iNOS, (B) IL-6 and (C) STAT3 are reduced by *L. paracasei* EM025-11 gavage of BALB/c mice with DSS-induced colitis. Control group - 2% Non-fat milk. One-way ANOVA, post-hoc, *p<0.05; **p<0.01; ***p<0.0001. n=8-10 mice/DSS groups, n=5 mice/Control groups.

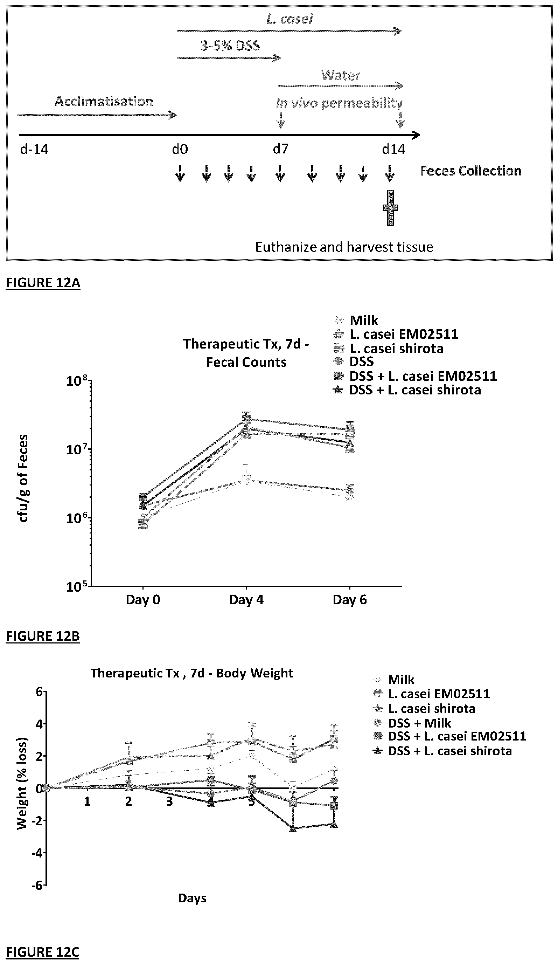

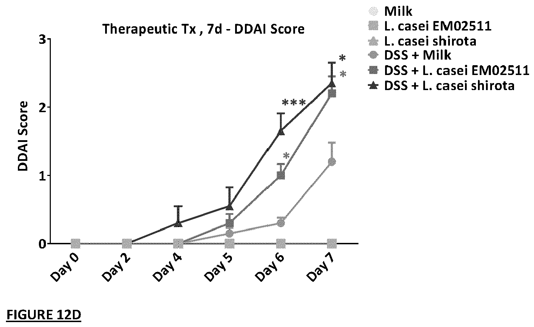

**Figure 12**: Colon mRNA expression of the (A) tight junction protein occludin, (B) adherence junction protein E-cadherin, (C) anti-microbial peptide lysozyme and (D) mucus production MUC2 are altered by *L. paracasei* EM025-11 gavage of BALB/c mice with DSS-induced colitis. Control group - 2% Non-fat milk. One-way ANOVA, post-hoc, *p<0.05; **p<0.01; ***p<0.0001. n=8-10 mice/DSS groups, n=5 mice/Control groups.

**Figure 13**: Growth of Lactobacillus sp. strains with different carbohydrates in mMRS

## Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

## Definitions and general preferences

Where used herein and unless specifically indicated otherwise, the following terms are intended to have the following meanings in addition to any broader (or narrower) meanings the terms might enjoy in the art:

Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

EP 4 070 670 A1

Disease and Therapy

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent (i.e. bacterium or composition of the invention) defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

"Metabolic disorder" refers to pre-diabetes, diabetes; Type-1 diabetes; Type-2 diabetes; metabolic syndrome; obesity; diabetic dyslipidemia; hyperlipidemia; hypertension; hypertriglyceridemia; hyperfattyacidemia; hypercholerterolemia; and hyperinsulinemia.

"Inflammatory disorder of the gut" refers to chronic inflammatory disorders of the gastrointestinal tract, including Inflammatory bowel diseases (IBD) such as Cronh's disease and ulcerative colitis. Symptoms of IBD include frequent bloody diarrhoea, abdominal cramping, anorexia, abdominal distension, and emesis.

"Inflammatory disorder" refers to a disease or condition characterised by the subjects' immune system attacking the body's own cells or tissue resulting in chronic pain, redness, swelling, stiffness and damage to normal tissues. Examples include rheumatoid arthritis, Gout, Lupus, Inflammatory bowel disease, Vasculitis, Myositis, Sclerodema, Ankylosing Spondylitis and Sjogren's Syndrome.

"Allergic disease" results from IgE-mediated immune responses to foreign protein (allergens). The majority of such reactions are IgE-mediated (type I) reactions. Individuals who develop such reactions are allergic. Those predisposed on a genetic basis to synthesize IgE to environmental allergens are atopic. Most allergic reactions are precipitated when a specific allergen aggregates several IgE molecules attached to IgE receptors on the surfaces of mast cells and basophils. Chemical mediators are released which lead to the immediate signs and symptoms associated with allergic diseases including hives, asthma, and anaphylaxis (Mekori et al. Crit Rev Food Sci Nutr. 996;36 Suppl:S1-18). Allergic diseases include asthma, allergy in children, atopic eczema, drug allergy, food allergy, rhinitis, and skin allergies.

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

*Lacticaseibacillus paracasei* EM205-11 strain

"*Lacticaseibacillus paracasei* EM205-11 strain" refers to the strain of bacteria deposited with the National Collection of Industrial and Marine Bacteria (Ferguson Building,

Craibstone Estate, Bucksburn, Aberdeen AB219YA, UK) under the Accession No. NCIMB 43703 on 17 December 2020. The Deposit was made by University College Cork, having an address of UCC, Cork, Ireland. The term is intended to include the strain in a viable or non-viable form, or mixtures of viable and non-viable bacteria. The strain may be provided in any format, for example as a liquid culture (or supernatant derived from a liquid culture), or cell material or extract derived from the strain or a culture of the strain, or in a dried or freeze-dried format. The invention may also employ growth media in which the strain of the invention was grown, or cell lysates generated using the strain of the invention. The term also includes mutants and variants of the deposited strain that are substantially identical, genetically and phenotypically, to the deposited strain and retain the activity of the deposited strain. Thus, the term includes derivatives of the strain that have been genetically engineered to modify the genome of the strain, typically without fundamentally altering the functionality of the organism, for example engineered for heterologous expression of a nucleic acid, or engineered to overexpress an endogenous gene, or engineered to silence a gene, to produce a recombinant or transformed strain of the invention. Genetic modifications may be carried out using recombinant DNA techniques and reagents, including gene editing technologies such as CRISP-Cas9 techniques (see below). The term also includes variants of the strain having natural or spontaneous genetic alterations. The term is also intended to encompass variant strains obtained by serial passage of the isolated strain of the invention. The variant generally comprises a gene that expresses a levanase protein, especially a levanase protein of SEQUENCE ID NO. 1 or a variant that is identical or substantially identical with SEQUENCE ID NO: 1, for example at least 70%, 80%, 90%, 91%, 92%, 93%, 94%, 95%, 96%, 97%, 98%, 99%, 99.1%, 99.2%, 99.3%, 99.4%, 99.5%, 99.6%, 99.7%, 99.8% or 99.9% identical with SEQUENCE ID NO:1. Sequence homology can be determined using an online homology algorithm "BLAST", publicly available at http://www.ncbi.nlm.nih.gov/BLAST/.

## Table 1

SEQUENCE ID NO: 1>Lactobacillus_casei_EM025-11_levanaseAA
MEMDEKKHYKMYKSKSVWVFACLSTCLIVSFFNDGQNVSAATSASSTQISQTNTGSQPNNE
TTGETAQSSVNSTATASSSSVADLPSSSDSKSSIGSTISQPAVDKKETSKSDTADNDLIKS
VTTSDSDKALPTSKTTLPTSNEQVQSSVGQSQTDQPASSATIATNAVTSDVSQNEQYNEPY
RNQYHYSSSQNWINDPNGLFYDSKTGLYNLYYQYNPEGNQWGNMSWGHAVSKDLINWTQED
VAIPMLQNQGWEDFTYTNTTGSLKDKGEVRYVGVPTTNWGDADGKKAIFSGSIVVDTNNVS
GLGKDAILAFYTADYQIATRKNDGAEDGWGTWIGLTEIQEQHLAYSLDGGKTFIQYSKDGN
AANPQAIIPTSMNQGGDAANFRDPSVVYDAVNKQYYLTVVSGQQALIYKSSNLLDWTYASK
IERENDVGNGVWECPSLVPMKVAGTNETKWVFCISVQQGAHATGSGMQYYVGNMTADGTWV

PESSKTLQNPMTMDSGEDFYAGIPFSNMPDGRTVMLAWQSNWSYVDEAKTSPWSGNMTLPR
ELSLKKNADTTDGYLLTNTVVKEIANNEEANVINKAESNFTVSRSDEQVQYEGKQYKISAT
FSWDEADKPKSVGFKLRVSDDQKYDMIVGYDLTTGLLYVQRLNTGEPNMGAPRDKMNATVN
ADGSITITVYVDETSIEAFANDGEKSITQNFFMRPENIGDQATTGVYVYSNDGTTKISDLT
INPITSIWNSTGQLTEKFVDENGNTIASDKIQTGRVGQSYTSESATIPGYVFVKENTDHIN
SNQLYTTQNQTITYTYRASQASVVTKDTTLVAGPSAAWNAADNLVGATDADGNALAVSDLT
VNGAVDPKTPGTYTVTYSYTDATDNKISKKATVTVIASKADIVTKDTTMVAGPSATWNAVD
NFVEATGADGSVLTLSDLTVNGAVDPKTPGTYTVTYSYTDAAGNKISKEATVTVIASKADI
VTKDTTMVAGASTIWNAADNFVEAKNADGNALTVSDLMINGTVDSKTPGTYTVTYSYTDAA
GNKISKEATVTVIASKADIVTKDTTMVAGPSATWNAADNLVIATDAKGNALALSDLTVNGA
VDPKTPGTYTVTYSYTDPAGNKISKEATVTVIASKADIVTKDTTMVAGPSAAWNAANNLVS
ATDADGNALAMSNLTVTGTVDLKTQGAYTVTYTYTDVAGNKISKEATVTVLTEKETNIEDN
TGSSISNDRENPPASITGKGGDDIHQNAKTTMTKKKTETLPQAGNHVNELAIVLGQMILAI
CVGGILWLKRRVKRV

## Compositions and Foods

The invention also relates to a composition comprising a strain of the invention. The composition may be a pharmaceutical composition, or a food composition, or a dietary supplement composition. The term "food" refers to a man-made food product including beverages, food additives and food supplements. Examples of foods include dairy products such as milk, yoghurt, cheese, cheese food, dairy powders, probiotic formulations, infant formula powders, follow-on milk formula, food for special medicinal purposes, meat products, soups, vegetable products, grain-based products containing inulin, plant-based dairy-like or plant-based dairy analog containing inulin, fruit juices, fruit products, breads, confectionary, cakes, sports supplements, nutritional supplements and the like.

In one embodiment, the composition includes a probiotic material. In one embodiment, the composition comprises a prebiotic material.

"Probiotic" refers to a microbial cell preparation, typically a bacterial cell preparation, that exerts a beneficial effect on the health or well-being of a host. They are described in Salminen et al (Trends Food Sci. Technol. 1999:10 107-110).

"Prebiotic" refers to a material or composition that can promote the growth of probiotic microbes or bacteria, especially bacterial growth in the mammalian gastrointestinal tract. Examples include oligosaccharides, dietary fibres, soluble dietary fibres, or mixtures thereof. Exemplary prebiotics are described in WO2011/039176, pages 12 to 15. Prebiotics are also described in Hutkins et al. ("Prebiotics: why definitions matter". Curr Opin Biotechnol. 37: 1-7. doi:10.1016/j.copbio.2015.09.001) and Gibson et al. ("Dietary modulation of the human colonic microbiota: introducing the concept of prebiotics". J. Nutr. 125 (6): 1401-1412.). In one embodiment, the prebiotic is an inulin carbohydrate.

"Soluble dietary fiber" or "soluble dietary fibers" are a composition that is formed chiefly of dextrose oligomers (i.e., oligosaccharide content having at least 98% dextrose monomeric residues, and degree of polymerization in the range of 2-30) optionally together with dextrose (i.e., monosaccharide). As used herein, the total amount of dextrose oligomers and dextrose in a soluble dietary fiber is at least 95%, desirably at least 98%. The total amount of oligomeric sugar alcohol residues in the soluble dietary fiber is no more than 2%, e.g., no more than 1% or even no more than 0.5%. Thus, the soluble dietary fiber is not a "polydextrose" as the term is commonly understood. Soluble dietary fibers can have a variety of molecular weights (consistent with remaining substantially water-soluble). As used herein, in many end uses, a relative viscosity is desired, and so it can be desirable for a soluble dietary fiber to have a relatively low molecular weight. In certain embodiments as otherwise described herein, the fiber has a weight average molecular weight in the range of 1000 g/mol to 2500 g/mol. For example, in certain such embodiments, the soluble dietary fiber has a weight average molecular weight in the range of 1000 g/mol to 2000 g/mol. In various additional embodiments as otherwise described herein, the soluble dietary fiber has a weight average molecular weight in the range of 1000 to 2250 g/mol, or 1000 g/mol to 1800 g/mol, or 1000 g/mol to 1600 g/mol, or 1200 to 2500 g/mol, or 1200 to 2250 g/mol, or 1200 g/mol to 2000 g/mol, or 1200 g/mol to 1800 g/mol, or 1200 g/mol to 1600 g/mol, or 1400 to 2500 g/mol, or 1400 to 2250 g/mol, or 1400 g/mol to 2000 g/mol, or 1400 g/mol to 1800 g/mol, or 1600 to 2500 g/mol, or 1600 to 2250 g/mol, or 1600 g/mol to 2000 g/mol, or 1800 g/mol to 2500 g/mol, or 1800 to 2250 g/mol, or 2000 g/mol to 2500 g/mol. As used herein, molecular weight of soluble dietary fibers are determined by gel permeation chromatography, using narrow standard pullulans as standards. In certain embodiments as otherwise described herein, the soluble dietary fiber comprises certain amounts of mono- and/or disaccharides. This will typically be chiefly dextrose and dextrose disaccharides such as maltose and isomaltose, but the person of ordinary skill in the art will appreciate that minor amounts of other mono- and/or disaccharides may be present. In certain embodiments as otherwise described herein, the total amount of mono- and disaccharides is up to 25 wt% on a dry solids basis, e.g., up to 20 wt%, or up to 15 wt%. In certain embodiments, a soluble dietary fiber as otherwise described herein can have a relatively lower amount of mono- and disaccharides, e.g., no more than 15 wt%, no more than 10 wt%. In some embodiments, the soluble dietary fiber has no more than 5 wt% total mono- and disaccharides. In certain embodiments as otherwise described herein, there is a relatively significant amount of mono- and/or disaccharides in the soluble dietary fiber. For example, in certain embodiments as otherwise described herein, the total amount of mono- and disaccharides is in the range of 3 wt% to 25 wt%. For example, in certain such embodiments, the total amount of mono- and disaccharides is in the range of 3 wt% to 20 wt%, or 5 wt% to 20 wt%, or 10 wt% to 20 wt%, or 15 wt% to 25 wt% on a dry solids basis.

"Synbiotic" as applied to a composition refers to a composition that comprises a probiotic bacterium and a prebiotic material or composition, that beneficially affects the host by improving the survival and activity of beneficial microorganisms in the gut. A synergistic synbiotic composition refers to a composition in which the prebiotic is a substrate for the probiotic bacterium.

The invention also relates to pharmaceutical compositions which comprises a pharmaceutically acceptable carrier. As used herein, the term "pharmaceutical composition" refers to a therapeutically effective amount of the strain of the invention, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. In the case of the present invention, the term "therapeutically effective amount" should be taken to mean an amount of therapeutic which results in a clinically significant increase in proliferation of target cells, for example gut epithelial cells or skin epithelial cells.

As used herein, the term "adjuvant" means an agent that enhances the recipient's immune response to an immunogenic peptide or protein. Details of suitable adjuvant compositions are well known to those skilled in the art.

As used herein, the term "pharmaceutically acceptable carrier" refers to a diluent, adjuvant, excipient, or vehicle with which the Therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Pharmaceutical compositions formulated or configured for oral administration and gastric transit are known in the art and include those described below:

- Sathish et al (Int. J. Pharm. Sci.2013; 258-269);
- Kushal et al (Int. Res. J. Pharm. 2013, 4(3));
- Philip et al (Oman Med J. 2010, 25(2));
- Polymers for controlled drug delivery - Peter Tarcha (CRC Press, 21 November 1990);
- Pharmaceutical coating technology - Michael Aulton et al (Taylor & Francis 27 October 1995);
- http://www.slideshare.net/Balimusale/oral-controlled-drug-delivery-system;
- European Patent No: 2418968 (Teagasc); and
- European Patent No: 2097072 (RCSI).
- Brayden et al. (European Journal of Pharmaceutical Sciences 79 (2015), 102-111.
- Tambuwala et al. (Journal of Controlled Release 217 (2015) 221-227.
- Zhang et al. Evaluation of alginate-whey protein microcapsules for intestinal delivery of lipophilic compounds in pigs (J. Sci. Food Agric. (2015).
- Lamprecht et al. (Journal of Controlled Release 104 (2005) 337-346.
- Hua et al. (Nanomedicine: Nanotechnology, Biology and Medicine 11 (2015) 1117-1132.
- Drug Delivery: Fundamentals and Applications (Chapter 7, Oral Drug Delivery, Hillary and Brayden)

As used herein, the term "food" refers to a man-made food product including beverages, food additives and food supplements. Examples of foods include dairy products such as milk, yoghurt, cheese, cheese food, dairy powders, probiotic formulations, infant formula powders, follow-on milk formula, food for special medicinal purposes, meat products, soups, vegetable products, fruit juices, fruit products, breads, confectionary, cakes, sports supplements, nutritional supplements and the like.

Dosage

It is preferable that the strain or composition is administered at least one per week over at treatment period of at least 4 weeks, and preferably at least 5, 6, 7, 8, 9, 10, 11, 12, 14, 16, 18 or 20 week period. Preferably, the strain or composition is administered several times a week, and ideally once a day. Compositions of the invention generally comprise between $10^3$ and $10^{12}$ cfu of the strain of the invention per gram of dry weight of the composition. In one embodiment, the composition comprises $10^3$ and $10^{12}$ cfu, or $10^4$ and $10^{12}$ cfu, or $10^6$ and $10^{10}$ cfu of the strain of the invention per gram of dry weight of the composition. A daily dose generally comprises between $10^3$ and $10^{12}$ cfu of the strain. In one embodiment, the daily dose comprises $10^3$ and $10^{12}$ cfu, or $10^4$ and $10^{12}$ cfu, or $10^6$ and $10^{10}$ cfu of the strain.

Recombinant DNA techniques and reagents

The invention also relates to mutants and variants of the strain that are substantially identical to the deposited strain and exhibit the same or similar gut health or anti-inflammatory functionality. This includes strains that are genetically engineered to alter the genome of the deposited strain (i.e. strained engineered for heterologous expression of nucleic acid), and variants obtained through natural genetic alterations such as spontaneous mutations, adaption and serial passage the term "engineered" as applied to a cell means genetically engineered using recombinant DNA technology, and generally involves the step of synthesis of a suitable expression vector (see below) and then transfecting (i.e. stably or transiently) the expression vector into a host cell (generally stable transfection). The term "heterologous expression" refers to expression of a nucleic acid in a host cell that does not naturally have the nucleic acid. Insertion of the nucleic acid into the heterologous host is performed by recombinant DNA technology. The term "heterologous in-situ expression" as applied to a bacterium of the invention means that the bacterium is capable of expressing the nucleic acid in-situ in the mammalian gut, especially in-situ expression when adhered to the epithelial layer of the gut. As used herein, the term "recombinant bacterium" or "transformed bacterium" refers to a bacterium comprising an exogenous nucleic acid stably integrated into the cellular genome. In another embodiment, the present invention provides a cell comprising a non-integrated (i.e. episomal) exogenous nucleic acid, such as a plasmid, cosmid, phagemid, or linear expression element, which comprises a sequence coding suitable for expression of an exogenous nucleic acid. In other embodiments, the present invention provides a cell line produced by stably transfecting a host cell with a plasmid comprising an expression vector of the invention.

As used herein, the term "expression vector" may be any suitable vector, including chromosomal, non-chromosomal, and synthetic nucleic acid vectors (a nucleic acid sequence comprising a suitable set of expression control elements) suitable for heterologous expression of a nucleic acid. Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. In one embodiment, the Tad pilus encoding nucleic acid molecule is comprised in a naked DNA or RNA vector, including, for example, a linear expression element (as described in, for instance, Sykes and Johnston, Nat Biotech 12, 355-59 (1997)), a compacted nucleic acid vector (as described in for instance U.S. Pat. No. 6,077,835 and/or WO 00/70087), or a plasmid vector such as pBR322, pUC 19/18, or pUC 118/119. Such nucleic acid vectors and the usage thereof are well known in the art (see, for instance, U.S. Pat. No. 5,589,466 and U.S. Pat. No. 5,973,972). In one embodiment, the DNA comprises an expression control sequence.

In one embodiment, the vector is suitable for heterologous expression of a nucleic acid in a bacterial cell. Examples of such vectors include expression vectors such as BlueScript (Stratagene), pIN vectors (Van Heeke & Schuster, 1989, J Biol Chem 264, 5503-5509), pET vectors (Novagen, Madison, Wis.) and the like. In one embodiment, the expression vector may also or alternatively be a vector suitable for expression in a yeast system. Any vector suitable for expression in a yeast system may be employed. Suitable vectors include, for example, vectors comprising constitutive or inducible promoters such as yeast alpha factor, alcohol oxidase and PGH (reviewed in: F. Ausubel et al., ed., 1987, Current Protocols in Molecular Biology, Greene Publishing and Wiley InterScience New York; and Grant et al., 1987, Methods in Enzymol 153, 516-544). In other embodiments, the expression vector is suitable for expression in baculovirus-infected insect cells. (Kost, T; and Condreay, J P, 1999, Current Opinion in Biotechnology 10 (5): 428-33.)

Expression control sequences are engineered to control and drive the transcription of genes of interest, and subsequent expression of proteins in various cell systems. Plasmids combine an expressible gene of interest with expression control sequences (i.e. expression cassettes) that comprise desirable elements such as, for example, promoters, enhancers, selectable markers, operators, etc. In an expression vector of the invention, Tad pilus-encoding nucleic acid molecules may comprise or be associated with any suitable promoter, enhancer, selectable marker, operator, repressor protein, polyA termination sequences and other expression-facilitating elements.

"Promoter" as used herein indicates a DNA sequence sufficient to direct transcription of a DNA sequence to which it is operably linked, i.e., linked in such a way as to permit transcription of the exogenous nucleotide sequence when the appropriate signals are present. The expression of a nucleotide sequence may be placed under control of any promoter or enhancer element known in the art. Examples of such elements include strong expression promoters (e.g., human CMV IE promoter/enhancer or CMV major IE (CMV-MIE) promoter, as well as RSV, SV40 late promoter, SL3-3, MMTV, ubiquitin (Ubi), ubiquitin C (UbC), and HIV LTR promoters). In some embodiments, the vector comprises a promoter selected from the group consisting of SV40, CMV, CMV-IE, CMV-MIE, RSV, SL3-3, MMTV, Ubi, UbC and HIV LTR.

Nucleic acid molecules of the invention may also be operably linked to an effective poly (A) termination sequence, an origin of replication for plasmid product in E. coli, an antibiotic resistance gene as selectable marker, and/or a convenient cloning site (e.g., a polylinker). Nucleic acids may also comprise a regulatable inducible promoter (inducible, repressable, developmentally regulated) as opposed to a constitutive promoter such as CMV IE (the skilled artisan will recognize that such terms are actually descriptors of a degree of gene expression under certain conditions).

Selectable markers are elements well-known in the art. Under the selective conditions, only cells that express the appropriate selectable marker can survive. Commonly, selectable marker genes express proteins, usually enzymes, that confer resistance to various antibiotics in cell culture. In other selective conditions, cells that express a fluorescent protein marker are made visible, and are thus selectable. Embodiments include beta-lactamase (bla) (beta-lactam antibiotic resistance or ampicillin resistance gene or ampR), bls (blasticidin resistance acetyl transferase gene), bsd (blasticidin-S deaminase resistance gene), bsr (blasticidin-S resistance gene), Sh ble (Zeocin® resistance gene), hygromycin phosphotransferase (hpt) (hygromycin resistance gene), tetM (tetracycline resistance gene or tetR), neomycin phosphotransferase II (npt) (neomycin resistance gene or neoR), kanR (kanamycin resistance gene), and pac (puromycin resistance gene).

In certain embodiments, the vector comprises one or more selectable marker genes selected from the group consisting of bla, bls, BSD, bsr, Sh ble, hpt, tetR, tetM, npt, kanR and pac. In other embodiments, the vector comprises one or more selectable marker genes encoding green fluorescent protein (GFP), enhanced green fluorescent protein (eGFP), cyano fluorescent protein (CFP), enhanced cyano fluorescent protein (eCFP), or yellow fluorescent protein (YFP).

For the purposes of this invention, gene expression in eukaryotic cells may be tightly regulated using a strong promoter that is controlled by an operator that is in turn regulated by a regulatory protein, which may be a recombinant "regulatory fusion protein" (RFP). The RFP consists essentially of a transcription blocking domain, and a ligand-binding domain that regulates its activity. Examples of such expression systems are described in US20090162901A1, which is herein incorporated by reference in its entirety.

As used herein "operator" indicates a DNA sequence that is introduced in or near a gene in such a way that the gene may be regulated by the binding of the RFP to the operator and, as a result, prevents or allow transcription of the gene of interest, i.e. a nucleotide encoding a polypeptide of the invention. A number of operators in prokaryotic cells and bacteriophage have been well characterized (Neidhardt, ed., Escherichia coli and Salmonella; Cellular and Molecular Biology 2d. Vol 2 ASM Press, Washington D.C. 1996). These include, but are not limited to, the operator region of the LexA gene of E. coli, which binds the LexA peptide, and the lactose and tryptophan operators, which bind the repressor proteins encoded by the Lad and trpR genes of E. coli. These also include the bacteriophage operators from the lambda PR and the phage P22 ant/mnt genes, which bind the repressor proteins encoded by lambda cl and P22 arc. In some embodiments, when the transcription blocking domain of the RFP is a restriction enzyme, such as Notl, the operator is the recognition sequence for that enzyme. One skilled in the art will recognize that the operator must be located adjacent to, or 3' to the promoter such that it is capable of controlling transcription by the promoter. For example, U.S. Pat. No. 5,972,650, which is incorporated by reference herein, specifies that tetO sequences be within a specific distance from the TATA box. In specific embodiments, the operator is preferably placed immediately downstream of the promoter. In other embodiments, the operator is placed within 10 base pairs of the promoter.

In an exemplary cell expression system, cells are engineered to express the tetracycline repressor protein (TetR) and a protein of interest is placed under transcriptional control of a promoter whose activity is regulated by TetR. Two tandem TetR operators (tetO) are placed immediately downstream of a CMV-MIE promoter/enhancer in the vector. Transcription of the gene encoding the protein of interest directed by the CMV-MIE promoter in such vector may be blocked by TetR in the absence of tetracycline or some other suitable inducer (e.g. doxycycline). In the presence of an inducer, TetR protein is incapable of binding tetO, hence transcription then translation (expression) of the protein of interest occurs. (See, e.g., U.S. Pat. No. 7,435,553, which is herein incorporated by reference in its entirety.)

The vectors of the invention may also employ Cre-lox recombination tools to facilitate the integration of a gene of interest into a host genome. A Cre-lox strategy requires at least two components: 1) Cre recombinase, an enzyme that catalyzes recombination between two loxP sites; and 2) loxP sites (e.g. a specific 34-base pair by sequence consisting of an 8-bp core sequence, where recombination takes place, and two flanking 13-bp inverted repeats) or mutant lox sites. (See, e.g. Araki et al., 1995, PNAS 92:160-4; Nagy, A. et al., 2000, Genesis 26:99-109; Araki et al., 2002, Nuc Acids Res 30(19):e103; and US20100291626A1, all of which are herein incorporated by reference). In another recombination strategy, yeast-derived FLP recombinase may be utilized with the consensus sequence FRT (see also, e.g. Dymecki, S. M., 1996, PNAS 93(12): 6191-6196).

Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

Materials and Methods

*Catabolic activity*

The ability of various carbohydrates, including several commercially available carbohydrates sold as prebiotic ingredients, to support growth of lactobacilli strains, including *L. paracasei* EM025-11 strain, isolated from a healthy elderly subject, was evaluated.

The growth of the strains in mMRS supplemented with the different carbohydrates was assessed as previously described (McLaughlin et al., 2015, PMID: 25817019).

*L. paracasei* EM025-11 strain was able to utilise 7 of the 13 carbohydrates tested as sole carbohydrate source. *L. paracasei* strain EM025-11 was the only strain able to utilise 6 high molecular weight inulin carbohydrates (Orafti and Frutafit carbohydrates) (Fig 13). The utilisation of the 6 high molecular weight inulin carbohydrates was confirmed by HPAEC analysis.

*Bacterial strains*

The bacterial strains used in this study *L. paracasei* EM025-11, *L. casei Shirota*, *L. lactis ME1363* and *L. sakei LME1323* were sourced from Prof Paul O'Toole's laboratory. The adherent and invasive *E. coli* (AIEC) strain HM605 was sourced from Dr Silvia Melgar' laboratory. The medium used to grow the bacteria are - *L. paracasei* EM025-11, *L. casei Shirota and L. sakei* - MRS agar; *L. lactis ME1363* - MRS agar supplemented with 0.5% glucose; HM605 - LB supplemented with $50\mu$g/ml ampicillin.

*Animals*

BALB/c females (OlaHsD, Harlan (Envigo) UK) were imported at an age of 6-8 weeks. Animals were housed in groups in individually ventilated cages (IVCs) in groups of maximally 5 animals and acclimatised for 2 weeks before entering the study.

*Oral Gavage of probiotic strains and colonisation*

Mice were orally gavaged daily for up to 21 days with $10^8$-$10^9$cfu of *L. paracasei EM025-11* and *L. casei Shirota* in 0.2ml PBS or 2% Non-fat milk. Faecal samples were collected every 2-3 days for enumeration of bacteria.

*Induction of colitis using Dextran Sodium Sulphate (DSS)*

Induction of colitis with DSS was performed as previously described (Melgar et al., 2005, PMID: 15637179). Briefly, DSS was dissolved in the drinking water and supplemented to mice at a concentration of 3% (w/w). Mice drank DSS for 7 days followed by a recovery period of 7 days where mice drink regular water.. The clinical symptoms recorded daily are body weight, fur texture/posture, stool consistency, rectal bleeding and prolapse according to the colitis scoring sheet in Table 1.

**TABLE 1**

| *Score* | *0* | *1* | *2* | *3* |
|---|---|---|---|---|
| **Weight loss** | Up to 5% | 6% to 15% | 15% to 25% | More than 25% |
| **Posture** | Not hunched | Mildly hunched | Very hunched | |
| **Fur texture** | Smooth | Mildly scruffy | Very scruffy | |
| **Stool Consistency** | Normal pellet | Changed form of pellets | Loose stool | Diarrhoea |
| **Rectal bleeding** | No blood | Blood in faeces | Blood alone | |

*Faecal plating*

Faeces were collected in re-weighed Eppendorf's and weighed. From the weights the following calculation was performed per faecal sample.

$$Faecal\ weight\ in\ eppendorf - eppendorf\ weight = faecal\ weight$$

$$\frac{Faecal\ sample}{concentration\ of\ 0.1} = volume$$

$$volume * 1000 = PBS\ volume\ needed$$

The calculated volume of PBS was added to each Eppendorf and the faeces was crushed with the aid of a sterile pipette tip, vortexed and once the sediment was at the bottom of the Eppendorf, the supernatant was collected. 100μl of the supernatant was added to the top row of a 96 well flat bottom plate followed by serial dilutions. A 10μl aliquot of each dilution was separately spotted onto a MRS agar plate, incubated at 37°C for 48hrs and the number of colonies present per dilution were counted.

*Collection of plasma and colon samples*

On the last day of the experiment, animals were euthanized by cervical dislocation and blood collected in EDTA-containing tubes, whole blood centrifuged at 1500 rpm, 5min and plasma collected and stored at -80°C until further use. Colon was excised and length and total weight measured. The distal colon was separated in 3 pieces, the most distal 0.5cm was stored in RNA later for gene expression analysis, the rest (3cm distal) was longitudinally divided in 2 pieces, one piece was frozen for protein (MSD) analysis and the other piece rolled as "Swiss role", embedded in OCT and frozen in liquid nitrogen for histology purposes.

*Histology and scoring*

Distal colon sections (5μm) were stained with haematoxylin and eosin according to standard histological procedures. The sections were coded and blindly scored using a light microscope (Olympus BX51, Olympus, Germany). A histology score reflecting the infiltration of inflammatory cells and epithelial structure was used in an scale of 0 to 6 where 0 = no signs of damage; 1 = few inflammatory cells, no signs of epithelial degeneration; 2 = mild inflammation, few signs of epithelial degeneration; 3 = moderate inflammation, few epithelial ulcerations; 4 = moderate to severe inflammation, ulcerations in more than 25% of the tissue section; 5 = moderate to severe inflammation, large ulcerations of more than 50% of the tissue section; 6 = severe inflammation and ulcerations of more than 75% of the tissue section (Melgar et al., 2006, PMID: 17088072).

*Mesenteric lymph node (MLN) cells isolation and activation*

Mesenteric lymph nodes were dissected from mice orally gavaged with *Lactobacillus* sp. strains, crushed by using the plunger of a 1 mL syringe, cell suspension centrifuged at 1400 rpm for 5 minutes and the cell pellet diluted in Dulbecco's Modified Eagle's Medium (DMEM; Sigma)+10% heat-inactivated foetal bovine serum (FBS; Sigma) for determination of total number of viable MLN cells using the Trypan blue assay. MLNs ($2.5 \times 10^6$ cells/mL) were activated for 48hrs with lipopolysaccharide (LPS, 100ng/mL) or Phorbol 12-myristate 13-acetate (PMA, 500ng/mL)/Ionomycin (Iono, 50ng/mL), followed by collection of supernatant for analysis of cytokine secretion by MSD/ELISA.

*Splenocytes isolation and activation*

The spleens from mice orally gavaged with *Lactobacillus* sp. strains were harvested, crushed through a 70μm cell strainer using the plunger of a sterile disposable syringe, followed by centrifugation at 1,500 rpm for 5 minutes of the cell suspension, cell pellet was washed and resuspended in 1ml Tris-buffered ammonium chloride for red cell lysis and incubated for 2 minutes at room temperature. The cells were underlaid with 1ml FBS and centrifuged at 300g for 10 minutes, cell pellet was washed in DMEM+ 10% FBS and the total number of viable Splenocytes were determined using the Trypan blue assay. The splenocytes ($2.5 \times 10^5$ cells/mL)were activated for 48hrs with LPS (100ng/mL) and PMA (500ng/mL)/Iono (50ng/mL), followed by collection of supernatant for analysis of cytokine secretion by MSD.

*Meso Scale Discovery (MSD) platform*

The levels of the cytokines IFN-γ, IL-1β, IL-2, IL-4, IL-5, IL-8, IL-10, IL-12p70 and TNF-α secreted by human PBMCs exposed to *Lactobacillus* sp. strains and IL-1β, IL-6, mKC, IL-10 produced from murine splenocytes, MLNs, plasma and colons were quantified using an electro-chemiluminescence multiplex system Sector 2400 imager from Meso Scale Discovery (MSD) (Gaithersburg, MD, USA) where antibodies labelled with a Sulfo-tag emitted light upon electrochemical stimulation. Protocol was carried out according to manufacturer's instructions. At the end of the protocol, plates were read using an MSD Quickplex SQ 120 plate reader.

*ELISA*

Cytokine levels in culture supernatants from epithelial cells were determined using IL-8 and CCL20 ELISAs (R&D Systems). Protocol for running of samples was carried out according to manufacturer's instructions. Plates were read at an absorbance of 490 nm and protein conc. was calculated from the standard curve.

*Reverse Transcriptase (RNA extraction)*

RNA extraction was carried out using an RNeasy Mini kit (Qiagen). RNA was extracted from triplicate wells (for cell culture) or from distal colon tissue following the manufacturer's protocol. The RNA concentration was quantified using a Nanodrop ND-1000 spectrophotometer (Thermo Scientific, MA, USA). DNase treatment of samples was carried out using the Ambion Turbo DNA-free Kit (ThermoFisher Scientific), followed byre-quantification of RNA concentration by Nanodrop spectrophotometer.

*Complementary DNA synthesis (cDNA)*

Reverse transcription was carried out using 250 - 1000 ng of RNA cDNA synthesis was carried out in a Thermocycler (Applied Biosystems)..

*Real-time reverse transcription polymerase chain reaction (qRT-PCR)*

The Universal Probe Library Assay Design Centre (https://www.roche-applied-science.com/sis/rtpcr/upl/adc.jsp) was used to design PCR primers and probes. Primer sequences were sent to Eurofins Genomics for synthesis. The housekeeping gene β-actin was used to correct for variability in the starting total RNA. All reactions were performed in duplicates using 384-well plates on the LightCycler®480 System (Roche). Nuclease free water was included as negative control. Thermal cycling conditions were used as recommended by the manufacturer (Roche). Relative changes in gene expression determined from qRT-PCR experiments were calculated using the 2-ΔΔCt method (Livak & Schmittgen, 2001, PMID: 11846609).

*Isolation and stimulation of human peripheral blood mononuclear cells (PBMC)*

Human peripheral blood mononuclear cells were isolated from 6 healthy human male volunteers in accordance with the Ethics Committee of University College Cork (CREC). Nine mL of venous blood was collected in sterile EDTA vacutainers (BD), layered over with mono-Poly Resolving medium (MPBio, USA)) and centrifuged for 300g for 30 minutes. Cell solution were removed from the interface, washed in DMEM + 10% FBS+ 1% penicillin/streptomycin (Pen/Strep; Sigma) and PBMC viability and cell number was evaluated using the Countess™ automated cell counter (Invitrogen). PBMCs were resuspended in complete media at $1 \times 10^6$ cells/mL and stimulated for 20 hrs with *Lactobacillus* sp. strains . The TLR1/2 agonist, Pam3CSK4 was used as a positive control. Supernatants were collected and stored at -20°C until analysis for cytokine/chemokine production by MSD.

*Cell Culture of human epithelial cell line CaCO2BBe1 and co-culture with bacteria strains Cell culture:* Human C2BBe1 [clone of Caco-2] cells were obtained from the American Type Culture Collection (ATCC). Cells were grown in high-glucose DMEM +10% FBS supplemented with 1% Pen/Strep, and 0.01% transferrin (Sigma) at 37°C in a 5% $CO_2$ incubator. Cells were cultured in a T175 flask (Grenier Bio-One) and passaged at 70% confluency by incubating the cells in 0.25% Trypsin EDTA Media (Sigma) for 8-10 minutes, followed trypsin-inactivation with cell media and centrifugation at 1000rpm for 5 minutes to isolate live cells. Cells were counted with a haemocytometer and seeded at approximately $1 \times 10^6$ cells. Cells used in this study were between passage 52 and 60.

*Co-culture experiments with bacteria following challenge with pro-inflammatory cytokine cocktail:* C2BBe1 cells ($1 \times 10^5$ cells/well) were seeded in full culture medium until approx. 90% confluency was reached. Control wells were also seeded to be used for counting the density of cells present on the day of infection. Twelve to sixteen hrs prior to infection culture medium was removed and replaced with DMEM. Bacterial strains to be used were grown overnight for use on the day of infection. The cell numbers in the control wells were counted and used to calculate a multiplicity of infection (MOI) of 10:1 bacteria: epithelial cells. Bacterial overnight cultures were read at an $OD_{600}$ to estimate cell

numbers ($OD_{600}$ of 1 = 8.8 x $10^8$ CFU/ml). 1mL of the overnight culture was pelleted by centrifugation at 6000 x g followed by 3 times washing with PBS. Bacteria were resuspended in DMEM supplemented with 10% FBS to the appropriate concentration to obtain an MOI of 10:1. The resuspended bacteria was co-cultured with C2BBe1 cells for 3 hrs followed by 3 times washing with a 1% Pen/Strep in PBS solution to remove bacteria. Cells were incubated with DMEM supplemented with 10% FBS, 1% Pen/Strep and a pro-inflammatory cytokine cocktail [containing recombinant human IL-1$\beta$ (10ng/mL), IFN$\gamma$ (10ng/mL) and TNF$\alpha$ (20ng/mL)] for 6 and 24hrs post infection. At the indicated time points cells and supernatants were collected and the material was preserved at -20°C/-80°C for subsequent analysis RNA extraction, quantitative (q)RT-PCR, ELISA.

*Co-culture experiments with bacteria for gene expression analysis:* Cells and bacteria were prepared as described above. Bacteria was co-cultured with C2BBe1 cells for 1 hr, followed by wash and further incubation in full media for 6 hrs post infection and cells were collected and stored for subsequent analysis RNA extraction and qRT-PCR.

*Invasion Assay using Gentamycin treatment*

Gentamicin assays were performed as previously described (Glasser et al., 2001, PMID: 11500426). C2BBe1 cells (2 x $10^5$ cells/mL) were seeded in DMEM + 10% FBS and incubated at 37°C, 5% $CO_2$ for 24 hrs. Bacterial overnight cultures were prepared the day before as outlined above. Prior to infection bacteria were washed 3x in PBS and resuspended in antibiotic free culture media. The $OD_{600}$ of the culture was read and bacteria were added at an MOI of 10:1 (1 x $10^7$ bacterial cells/mL). Bacteria were co-cultured with IECs for 1 hr at 37°C, 5% $CO_2$, followed by 3x washes in PBS to remove extracellular bacteria. Fresh DMEM+10% FBS and supplemented with 50 $\mu$g/ml Gentamicin was added , incubated for 1 hr allowing gentamicin to kill the remaining extracellular bacteria. Cells were further maintained in DMEM+ 10% FBS and 50 $\mu$g/ml gentamicin for 6 hours at 37°C, 5% CO2 to allow for replication of internalised bacteria. Cells and supernatants were collected and preserved at -20°C/-80°C for subsequent analysis.

*Adherence assay*

Overnight cultures of *Lactobacillus* sp. strains and HM605, were prepared as described. Bacteria were washed 3x in PBS and dilutions were made to obtain an MOI of 100/1000/2000, added to C2BBe1 cells, incubated at 37°C for 1 hr, washed 3x in PBS, lysed by addition of Triton-X100 0.1% solution and the number of viable adhering bacteria was determined by plating serial dilutions on appropriate agar plates. Bacteria-C2BBe1 cocultures were stained with Giemza and imaged by light microscopy.

*In vitro barrier function assay measured by Transepithelial resistance (TER)*

Trans-epithelial electrical resistance (TER) was used to measure barrier integrity of the C2BBe1 cell monolayer in a 24-well transwell ThinCerts™ Tissue Culture Inserts (Cruinn). C2BBe1 cells were seeded (4x$10^5$ cells/insert) and TER readings followed the cells growing to confluency, with a plateau indicating the cells had formed a tight monolayer. TER measurements were taken every 2-3 days using an EVOMX resistance meter (World Precision Instruments) and the media changed at the same time. Once the TER plateau is reached, bacteria, grown as outlined, was diluted to an MOI of 10:1, added to the transwell insert and cultured for 3 hours. TER readings were taken and media from the lower well replaced either with a 10mM $H_2O_2$ solution or medium. TERs readings were taken at time zero (addition of $H_2O_2$) and every 2 hours for a total of 6 hours. TER reading are expressed as per cent of each treatment's initial value.

*Statistical Analysis*

GraphPad Prism 6.01 software was used to perform all statistical analysis. Unless otherwise stated all experiments were performed in triplicates. The students two tail t-Test was used to compare the differences between two groups of data. Differences between three or more groups were calculated by analysis of variance (ANOVA) followed by post hoc test. Differences were considered to be significant if $p \leq 0.05$.

Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

**BUDAPEST TREATY ON THE INTERNATIONAL RECOGNITION
OF THE DEPOSIT OF MICROORGANISMS FOR THE
PURPOSES OF PATENT PROCEDURE**

|  |  |
|---|---|
| University College Cork<br><br>Room 447 Food Science Building,<br>University College Cork, T12 K8AF<br>Cork, Ireland | **INTERNATIONAL FORM**<br><br>**RECEIPT IN THE CASE OF AN ORIGINAL DEPOSIT**<br>issued pursuant to Rule 7.1 by the<br>**INTERNATIONAL DEPOSITARY AUTHORITY**<br>identified at the bottom of this page |

NAME AND ADDRESS OF DEPOSITOR

| I. IDENTIFICATION OF THE MICROORGANISM | |
|---|---|
| Identification reference given by the DEPOSITOR: | Accession number given by the INTERNATIONAL DEPOSITARY AUTHORITY: |
| Lactobacillus paracasei<br><br>EM025-11 | NCIMB 43703 |

| II. SCIENTIFIC DESCRIPTION AND/OR PROPOSED TAXONOMIC DESIGNATION |
|---|
| The microorganism identified under I above was accompanied by: |
| A proposed taxonomic designation |

| III. RECEIPT AND ACCEPTANCE |
|---|
| This International Depositary Authority accepts the microorganism identified under I above (date of the original deposit)*, which was received by it on: |
| 17/12/2020 |

| IV. RECEIPT OF REQUEST FOR CONVERSION |
|---|
| The microorganism identified under I above was received by this International Depositary Authority on (date of original deposit) and a request to convert the original deposit to a deposit under the Budapest Treaty was received by it on (date of receipt of request for conversion). |

| V. INTERNATIONAL DEPOSITARY AUTHORITY | |
|---|---|
| Name & Address:<br>NCIMB Ltd. Ferguson Building, Craibstone Estate, Bucksburn, Aberdeen, AB21 9YA Scotland. | |
| Name, Signature and Date of person(s) having the power to represent the International Depositary Authority or of authorised official(s): | Sammi Wilson,    24/12/2020<br>Microbiologist |

*Where Rule 6/4(d) applies, such date is the date on which the status of International Depositary Authority was acquired.

Form BP/4 (sole page)

**Claims**

1. An isolated *Lacticaseibacillus paracasei* EM205-11 strain as deposited with the National Collection of Industrial and Marine Bacteria under the Accession No. NCIMB 43703 on 17 December 2020

2. A cell extract or supernatant of *Lacticaseibacillus paracasei* EM205-11 strain

3. A composition comprising the isolated *Lacticaseibacillus paracasei* EM205-11 strain of Claim 1 or a cell extract or supernatant of *Lacticaseibacillus paracasei* EM205-11 strain of Claim 2.

4. A composition according to Claim 3, selected from a food or beverage product or a nutritional supplement.

5. A composition according to Claim 3 or 4 which is a synbiotic composition comprising a prebiotic material.

6. A composition according to Claim 5 in which the prebiotic material comprises an inulin carbohydrate.

7. A composition according to any of Claims 3 to 6, in which the isolated *Lacticaseibacillus paracasei* EM205-11 strain is viable or non-viable.

8. A composition according to any of Claims 3 to 7, in which the *Lacticaseibacillus paracasei* EM205-11 strain extract is a cell lysate.

9. A composition according to any of Claims 3 to 8, comprising at least $10^6$ cfu per gram of composition.

**10.** A pharmaceutical composition comprising an isolated *Lacticaseibacillus paracasei* EM205-11 strain of Claim 1 or supernatant or extract of Claim 2, and a suitable pharmaceutical excipient.

**11.** An isolated *Lacticaseibacillus paracasei* EM205-11 strain of Claim 1 in a dried or lyophilised form.

**12.** A composition according to any of Claims 3 to 10, for use in a method of maintaining gut health in a subject.

**13.** A composition according to any of Claims 3 to 10, for use in a method of treating an inflammatory condition in a subject.

**14.** A composition according to any of Claims 3 to 10, for use in a method of treating or preventing an indication selected from:

Intestinal discomfort;
Bloating;
Constipation;
Leaky gut syndrome;
Irritable bowel syndrome (or a symptom thereof);
Inflammatory bowel disease (or a symptom thereof);
Ulcerative colitis;
Crohn's disease;
Metabolic syndrome; and
an allergic disease

**15.** A method selected from:

producing a supernatant from an isolated *Lacticaseibacillus paracasei* EM205-11 strain comprising a step of culturing the isolated strain and separating the supernatant from the strain; and
producing an extract from an isolated *Lacticaseibacillus paracasei* EM205-11 strain comprising a step of lysing the cell and separating the cell extract from lysed cell material.

## 1hr Co-culture

FIGURE 1A

## 1hr Co-culture + 5hr Gentamicin Treatment

FIGURE 1B

**FIGURE 1C**

**FIGURE 2A**

**IL8 24h**

**FIGURE 2B**

**CCL20 24h**

**FIGURE 2C**

EP 4 070 670 A1

EP 4 070 670 A1

**FIGURE 2D**

**FIGURE 2E**

## 6 Hours Transepithelial Resistance

**FIGURE 3A**

Muc3a

Glycocalyx associated

Muc5ac

Gel forming

**FIGURE 3B**

**FIGURE 3C**

FIGURE 4A

FIGURE 4B

FIGURE 4C

FIGURE 4E

FIGURE 4D

**CCL20**

**NFKBIA**

FIGURE 4F

FIGURE 4G

FIGURE 4H

EP 4 070 670 A1

FIGURE 5A

FIGURE 5B

FIGURE 5C

FIGURE 5D

FIGURE 5E

FIGURE 5F

FIGURE 5G

**IL4**

**IL13**

FIGURE 5H

FIGURE 5I

FIGURE 5J

FIGURE 5K

**FIGURE 6A**

**FIGURE 6B**

FIGURE 7A

FIGURE 7B

FIGURE 7C

FIGURE 7D

FIGURE 8A

FIGURE 8B

FIGURE 8C

FIGURE 8D

EP 4 070 670 A1

FIGURE 9A

FIGURE 9B

FIGURE 9C

FIGURE 9D

FIGURE 10A

FIGURE 10B

FIGURE 10C

**FIGURE 11A**

**FIGURE 11B**

**FIGURE 11C**

EP 4 070 670 A1

**FIGURE 12A**

**FIGURE 12B**

**FIGURE 12C**

FIGURE 12D

**Therapeutic Tx, 7d - Colon Lenght**

FIGURE 13A

**Therapeutic Tx , 7d- Proximal Colon**

FIGURE 13B

**Therapeutic Tx , 7d - Distal Colon**

FIGURE 13C

**Therapeutic Tx, 7d - *In vivo* Permeability**

FIGURE 13D

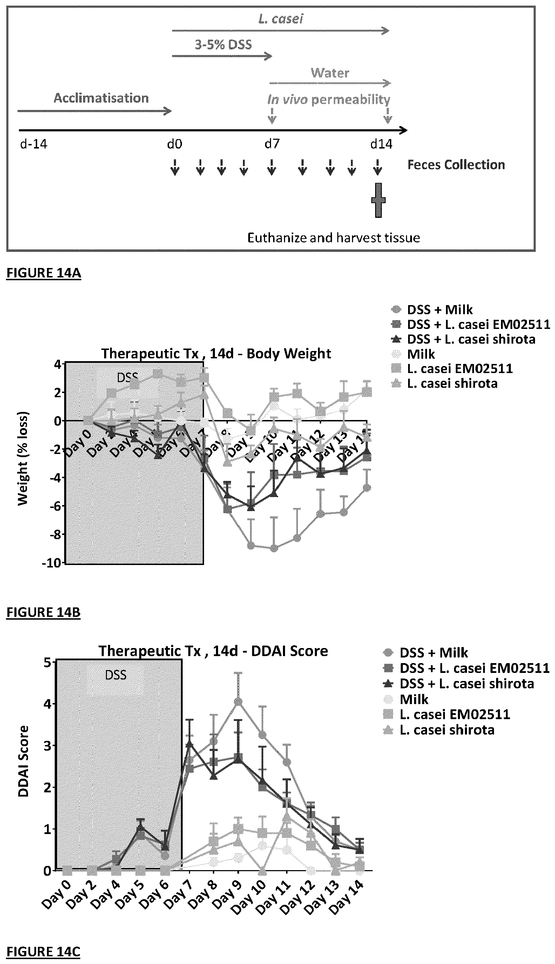

FIGURE 14A

FIGURE 14B

FIGURE 14C

**Therapeutic Tx, 14d - Colon Lenght**

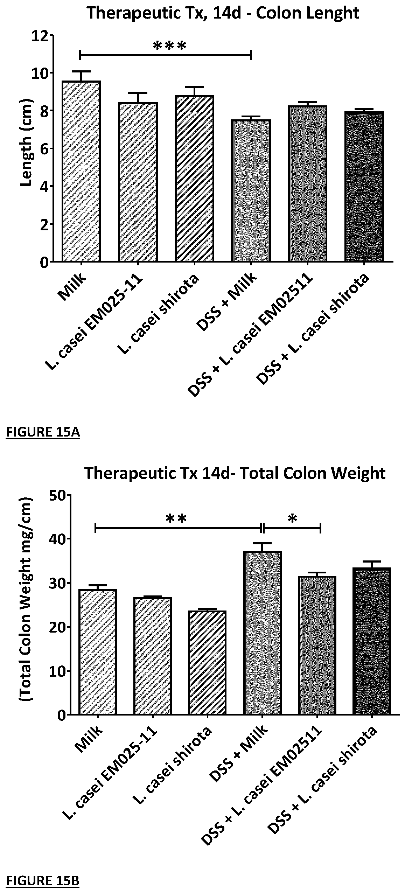

FIGURE 15A

**Therapeutic Tx 14d- Total Colon Weight**

FIGURE 15B

**Therapeutic Tx , 14d-  Proximal Colon**

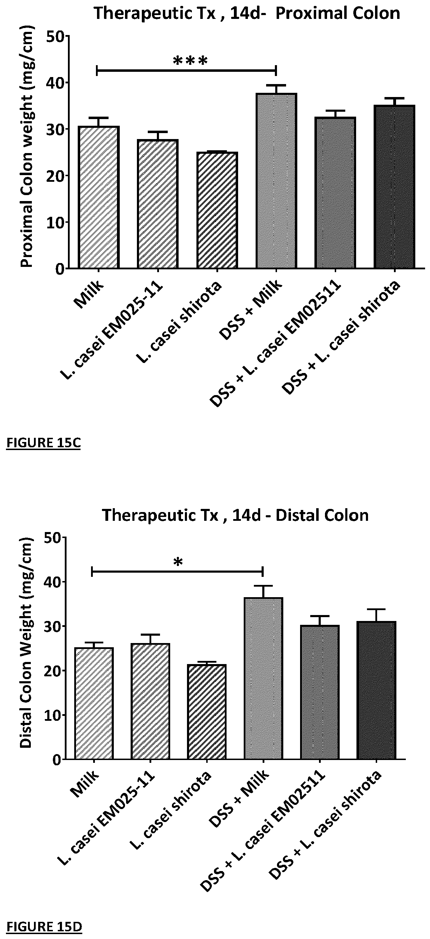

FIGURE 15C

**Therapeutic Tx , 14d - Distal Colon**

FIGURE 15D

Therapeutic Tx 14d- Histology

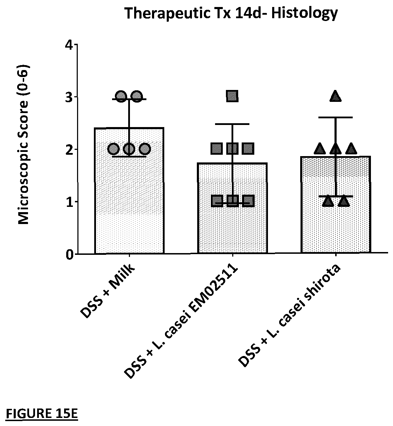

FIGURE 15E

## Therapeutic Tx 14d- Spleen

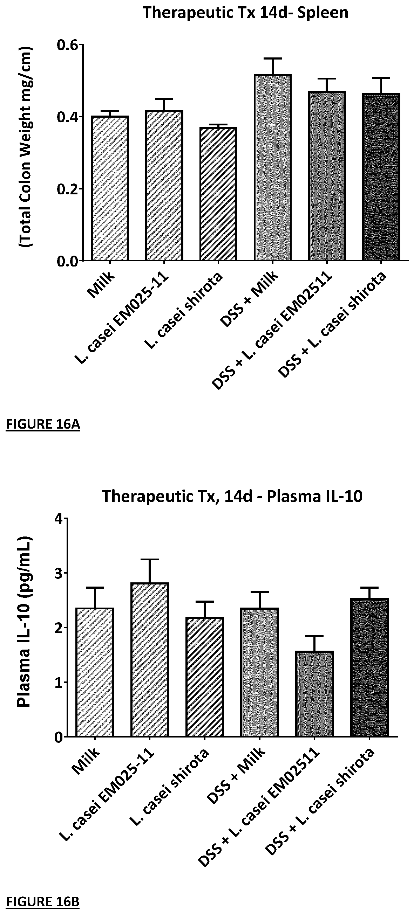

FIGURE 16A

## Therapeutic Tx, 14d - Plasma IL-10

FIGURE 16B

**Therapeutic Tx, 14d - Plasma IL-6**

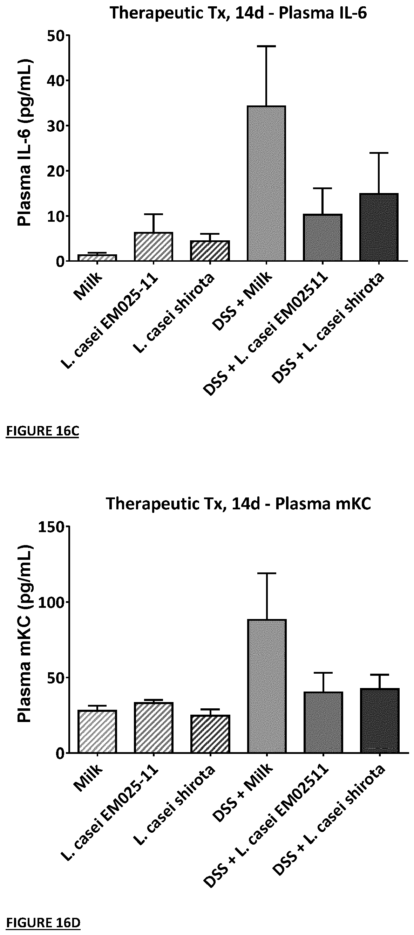

FIGURE 16C

**Therapeutic Tx, 14d - Plasma mKC**

FIGURE 16D

**Therapeutic Tx, 14d - Colon mKC**

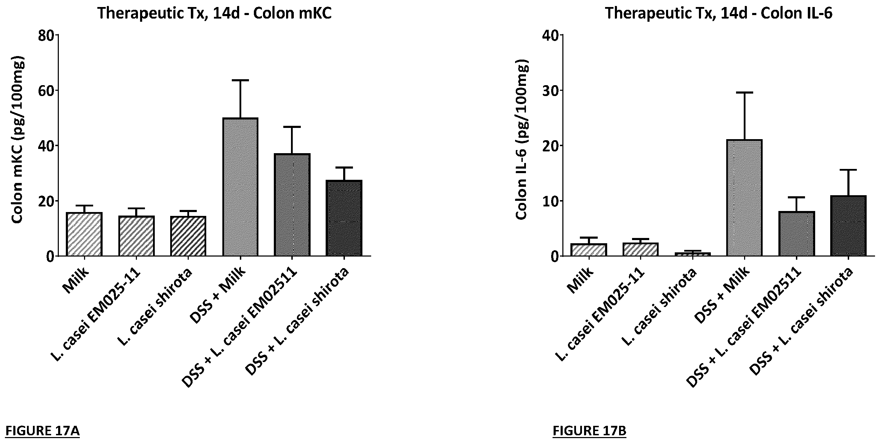

FIGURE 17A

**Therapeutic Tx, 14d - Colon IL-6**

FIGURE 17B

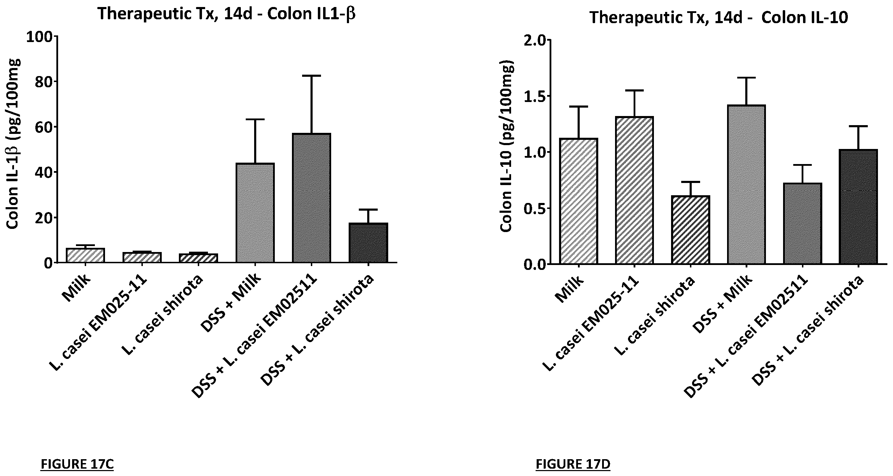

FIGURE 17D

FIGURE 17C

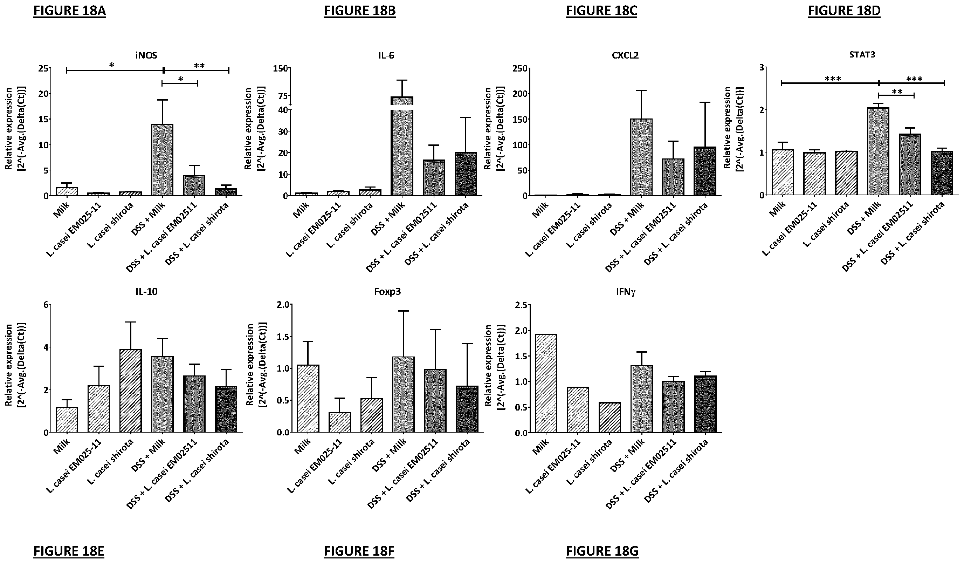

FIGURE 18A

FIGURE 18B

FIGURE 18C

FIGURE 18D

iNOS

IL-6

CXCL2

STAT3

IL-10

Foxp3

IFNγ

FIGURE 18E

FIGURE 18F

FIGURE 18G

EP 4 070 670 A1

60

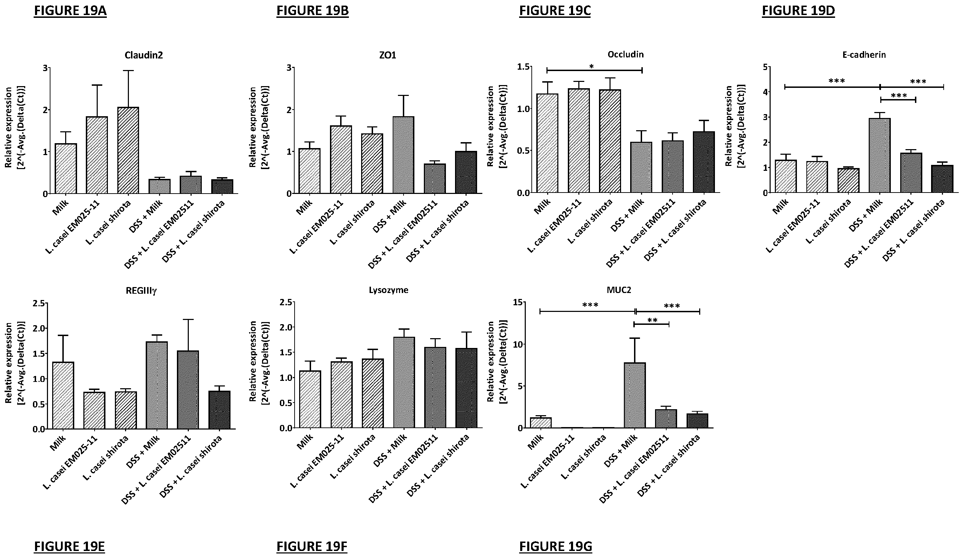

FIGURE 19A  FIGURE 19B  FIGURE 19C  FIGURE 19D  FIGURE 19E  FIGURE 19F  FIGURE 19G

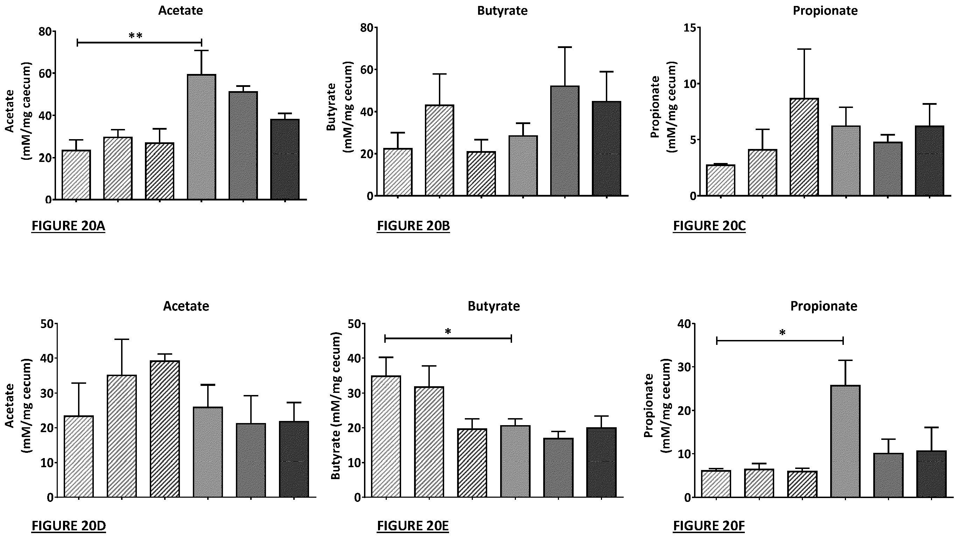

FIGURE 20A

FIGURE 20B

FIGURE 20C

FIGURE 20D

FIGURE 20E

FIGURE 20F

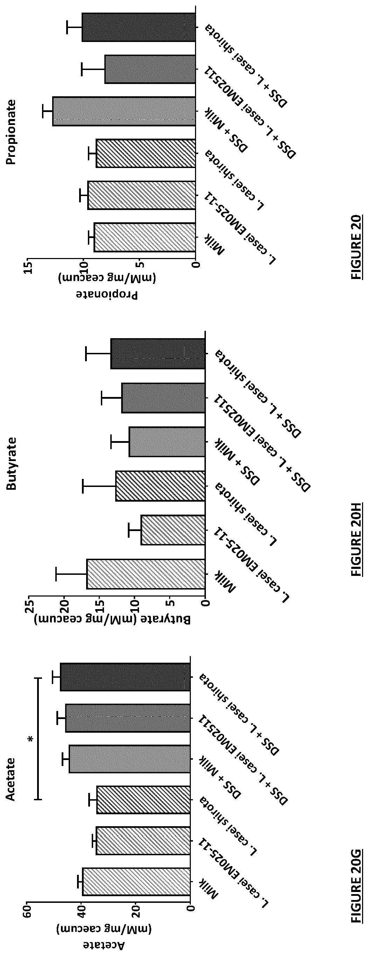

FIGURE 20

FIGURE 20H

FIGURE 20G

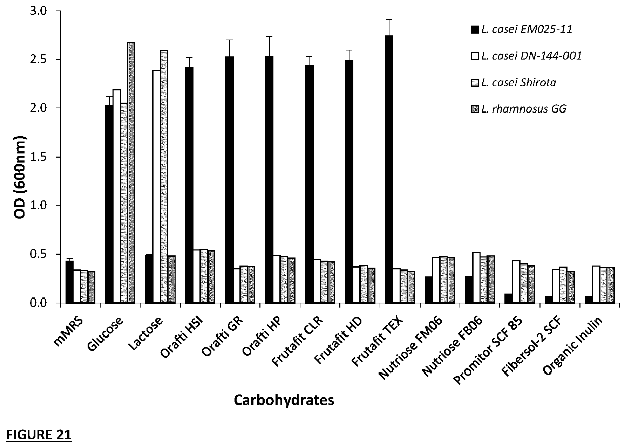

**FIGURE 21**

Europäisches
Patentamt

European
Patent Office

Office européen
des brevets

**EUROPEAN SEARCH REPORT**

Application Number

EP 21 16 7470

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 944 730 A (UNIV HUAZHONG AGRICULTURAL) 17 November 2020 (2020-11-17) | 2-6,8, 10,12-14 | INV. A23L33/135 A23L33/00 A61K35/747 A61P1/00 A61P3/10 A61P37/08 C12N1/20 C12R1/225 A61P37/02 |
| Y | * paragraphs [0007] - [0019], [0022], [0023]; claims 1-6; figure 3; examples 1,2,4 * | 1,7,9, 11,15 | |
| A | CN 107 916 236 A (BENED BIOMEDICAL CO LTD) 17 April 2018 (2018-04-17) * paragraphs [0005] - [0012]; claims 1-15; table 4 * | 1-15 | |
| X | TARRAH ARMIN ET AL: "Complete Genome Sequence and Carbohydrates-Active EnZymes (CAZymes) Analysis ofDTA72, a Potential Probiotic Strain with Strong Capability to Use Inulin", CURRENT MICROBIOLOGY, SPRINGER-VERLAG, NEW YORK, vol. 77, no. 10, 4 July 2020 (2020-07-04), pages 2867-2875, XP037229004, ISSN: 0343-8651, DOI: 10.1007/S00284-020-02089-X [retrieved on 2020-07-04] | 1-15 | |
| Y | * abstract * * page 2868, column 1, paragraph 2 - page 2869, column 1, paragraph 1 * * page 2872, column 1, paragraph 3 - page 2874, column 1, paragraph 1 * | 1,7,9, 11,15 | TECHNICAL FIELDS SEARCHED (IPC) A23L C12R A61K A61P C12N |

-/--

The present search report has been drawn up for all claims

| Place of search | Date of completion of the search | Examiner |
|---|---|---|
| Munich | 16 September 2021 | Schlegel, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GOH Y J ET AL: "Identification of a putative operon involved in fructooligosaccharide utilization by Lactobacillus paracasei", APPLIED AND ENVIRONMENTAL MICROBIOLOGY, AMERICAN SOCIETY FOR MICROBIOLOGY, US, vol. 72, no. 12, December 2006 (2006-12), pages 7518-7530, XP002582543, ISSN: 0099-2240, DOI: 10.1128/AEM.00877-06 [retrieved on 2006-10-06] * abstract * * page 7518 - page 7519, column 1, paragraph 3 * * page 7529, column 1, paragraph 2 * | 1-15 | |
| X | MAKRAS LEFTERIS ET AL: "Lactobacillus paracasei subsp. paracasei 8700:2 Degrades Inulin-Type Fructans Exhibiting Different Degrees of Polymerization", APPLIED AND ENVIRONMENTAL MICROBIOLOGY, vol. 71, no. 11, November 2005 (2005-11), pages 6531-6537, XP55841086, US ISSN: 0099-2240, DOI: 10.1128/AEM.71.11.6531-6537.2005 | 2-6,8, 10,12-14 | |
| Y | * page 6531; table 1 * * page 6533, column 1, paragraph 3 - column 2, paragraph 2 * * page 6534, column 1, paragraph 2 * * page 6534, column 2, paragraph 2 - page 6536, column 2, paragraph 1 * | 1,7,9, 11,15 | |
| Y | US 2012/225459 A1 (KELLY STEVEN LEWIS [GB]) 6 September 2012 (2012-09-06) * paragraphs [0003], [0029]; claims 1-32; sequence 3 * | 1,7,9, 11,15 | |

TECHNICAL FIELDS
SEARCHED      (IPC)

-/--

The present search report has been drawn up for all claims

| Place of search | Date of completion of the search | Examiner |
|---|---|---|
| Munich | 16 September 2021 | Schlegel, Birgit |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

........................................................................

& : member of the same patent family, corresponding
    document

EPO FORM 1503 03.82 (P04C01)

page 2 of 3

Europäisches
Patentamt

European
Patent Office

Office européen
des brevets

**EUROPEAN SEARCH REPORT**

Application Number

EP 21 16 7470

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MUELLER M ET AL: "PURIFICATION AND SUBSTRATE SPECIFICITY OF AN EXTRACELLULAR FRUCTANHYDROLASE FROM LACTOBACILLUS PARACASEI SSP. PARACASEI P 4134", NEW PHYTOLOGIST, WILEY-BLACKWELL PUBLISHING LTD, GB, vol. 136, no. 1, 1997, pages 89-96, XP000982638, ISSN: 0028-646X, DOI: 10.1046/J.1469-8137.1997.00725.X | 2-6,8, 10,12-14 | |
| Y | * abstract * * page 92, column 1, paragraph 4 - column 2, paragraph 1 * ----- | 1,7,9, 11,15 | |
| T | LEIDIANE A. A. MENEZES ET AL: "Effects of Sourdough on FODMAPs in Bread and Potential Outcomes on Irritable Bowel Syndrome Patients and Healthy Subjects", FRONTIERS IN MICROBIOLOGY, vol. 9, 21 August 2018 (2018-08-21), XP055510378, DOI: 10.3389/fmicb.2018.01972 * the whole document * ----- | 12-14 | |
| X | US 2021/077550 A1 (KO GWANG PYO [KR] ET AL) 18 March 2021 (2021-03-18) * claims 1-16; examples 1-7 * ----- | 1-4,7-15 | |

TECHNICAL FIELDS
SEARCHED        (IPC)

The present search report has been drawn up for all claims

| Place of search | Date of completion of the search | Examiner |
|---|---|---|
| Munich | 16 September 2021 | Schlegel, Birgit |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 16 7470

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2021

| Patent document cited in search report | | Publication date | Patent family member(s) | | Publication date |
|---|---|---|---|---|---|
| CN 111944730 | A | 17-11-2020 | NONE | | |
| CN 107916236 | A | 17-04-2018 | CN 107916236 A | | 17-04-2018 |
| | | | TW 201814039 A | | 16-04-2018 |
| US 2012225459 | A1 | 06-09-2012 | EP 2401368 A1 | | 04-01-2012 |
| | | | GB 2468136 A | | 01-09-2010 |
| | | | US 2012225459 A1 | | 06-09-2012 |
| | | | WO 2010097416 A1 | | 02-09-2010 |
| US 2021077550 | A1 | 18-03-2021 | AU 2019265196 A1 | | 26-11-2020 |
| | | | BR 112020022749 A2 | | 02-03-2021 |
| | | | CA 3099668 A1 | | 14-11-2019 |
| | | | CN 112534043 A | | 19-03-2021 |
| | | | EP 3792342 A1 | | 17-03-2021 |
| | | | JP 2021522867 A | | 02-09-2021 |
| | | | KR 20190129014 A | | 19-11-2019 |
| | | | KR 20200140224 A | | 15-12-2020 |
| | | | KR 20200140225 A | | 15-12-2020 |
| | | | PH 12020551878 A1 | | 31-05-2021 |
| | | | SG 11202010944R A | | 30-12-2020 |
| | | | US 2021077550 A1 | | 18-03-2021 |
| | | | WO 2019216662 A1 | | 14-11-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011039176 A
- EP 2418968 A
- EP 2097072 A
- US 6077835 A
- WO 0070087 A
- US 5589466 A
- US 5973972 A
- US 20090162901 A1
- US 5972650 A
- US 7435553 B
- US 20100291626 A1

### Non-patent literature cited in the description

- **MEKORI et al.** *Crit Rev Food Sci Nutr.,* vol. 996 (36), 1-18
- **SALMINEN et al.** *Trends Food Sci. Technol.,* 1999, vol. 10, 107-110
- **HUTKINS et al.** Prebiotics: why definitions matter. *Curr Opin Biotechnol.,* vol. 37, 1-7
- **GIBSON et al.** Dietary modulation of the human colonic microbiota: introducing the concept of prebiotics. *J. Nutr.,* vol. 125 (6), 1401-1412
- **SATHISH et al.** *Int. J. Pharm. Sci.,* 2013, 258-269
- **KUSHAL et al.** *Int. Res. J. Pharm.,* 2013, vol. 4 (3
- **PHILIP et al.** *Oman Med J.,* 2010, vol. 25 (2
- **PETER TARCHA.** Polymers for controlled drug delivery. CRC Press, 21 November 1990
- **MICHAEL AULTON et al.** Pharmaceutical coating technology. Taylor & Francis, 27 October 1995
- **BRAYDEN et al.** *European Journal of Pharmaceutical Sciences,* 2015, vol. 79, 102-111
- **TAMBUWALA et al.** *Journal of Controlled Release,* 2015, vol. 217, 221-227
- **ZHANG et al.** Evaluation of alginate-whey protein microcapsules for intestinal delivery of lipophilic compounds in pigs. *J. Sci. Food Agric.,* 2015
- **LAMPRECHT et al.** *Journal of Controlled Release,* 2005, vol. 104, 337-346
- **HUA et al.** *Nanomedicine: Nanotechnology, Biology and Medicine,* 2015, vol. 11, 1117-1132
- Drug Delivery: Fundamentals and Applications. **HILLARY ; BRAYDEN.** Oral Drug Delivery
- **SYKES ; JOHNSTON.** *Nat Biotech,* 1997, vol. 12, 355-59
- **VAN HEEKE ; SCHUSTER.** *J Biol Chem,* 1989, vol. 264, 5503-5509
- Current Protocols in Molecular Biology. Greene Publishing and Wiley InterScience, 1987
- **GRANT et al.** *Methods in Enzymol,* 1987, vol. 153, 516-544
- **KOST, T ; CONDREAY, J P.** *Current Opinion in Biotechnology,* 1999, vol. 10 (5), 428-33
- Escherichia coli and Salmonella. Cellular and Molecular Biology. ASM Press, 1996, vol. 2
- **ARAKI et al.** *PNAS,* 1995, vol. 92, 160-4
- **NAGY, A. et al.** *Genesis,* 2000, vol. 26, 99-109
- **ARAKI et al.** *Nuc Acids Res,* 2002, vol. 30 (19), e103
- **DYMECKI, S. M.** *PNAS,* 1996, vol. 93 (12), 6191-6196

SEQUENCE LISTING

<110>   UNIVERSITY COLLEGE CORK NATIONAL UNIVERSITY OF IRELAND CORK

<120>   Lacticaseibacillus paracasei EM205-11 and uses thereof

<130>   P13630EP00

<160>

<170>   PatentIn version 3.5

<210>   1
<211>   1296
<212>   PRT
<213>   Lactobacillus casei

<400>   1

Met Glu Met Asp Glu Lys Lys His Tyr Lys Met Tyr Lys Ser Lys Ser
1               5                   10                  15

Val Trp Val Phe Ala Cys Leu Ser Thr Cys Leu Ile Val Ser Phe Phe
                20                  25                  30

Asn Asp Gly Gln Asn Val Ser Ala Ala Thr Ser Ala Ser Ser Thr Gln
            35                  40                  45

Ile Ser Gln Thr Asn Thr Gly Ser Gln Pro Asn Asn Glu Thr Thr Gly
        50                  55                  60

Glu Thr Ala Gln Ser Ser Val Asn Ser Thr Ala Thr Ala Ser Ser Ser
65                  70                  75                  80

Ser Val Ala Asp Leu Pro Ser Ser Ser Asp Ser Lys Ser Ser Ile Gly
                85                  90                  95

Ser Thr Ile Ser Gln Pro Ala Val Asp Lys Lys Glu Thr Ser Lys Ser
                100                 105                 110

Asp Thr Ala Asp Asn Asp Leu Ile Lys Ser Val Thr Thr Ser Asp Ser
            115                 120                 125

Asp Lys Ala Leu Pro Thr Ser Lys Thr Thr Leu Pro Thr Ser Asn Glu
        130                 135                 140

Gln Val Gln Ser Ser Val Gly Gln Ser Gln Thr Asp Gln Pro Ala Ser
145                 150                 155                 160

Ser Ala Thr Ile Ala Thr Asn Ala Val Thr Ser Asp Val Ser Gln Asn
                165                 170                 175

```
Glu Gln Tyr Asn Glu Pro Tyr Arg Asn Gln Tyr His Tyr Ser Ser Ser
        180                 185                 190

Gln Asn Trp Ile Asn Asp Pro Asn Gly Leu Phe Tyr Asp Ser Lys Thr
        195                 200                 205

Gly Leu Tyr Asn Leu Tyr Tyr Gln Tyr Asn Pro Glu Gly Asn Gln Trp
        210                 215                 220

Gly Asn Met Ser Trp Gly His Ala Val Ser Lys Asp Leu Ile Asn Trp
225                 230                 235                 240

Thr Gln Glu Asp Val Ala Ile Pro Met Leu Gln Asn Gln Gly Trp Glu
                245                 250                 255
```

SEQUENCE LISTING

<110>   UNIVERSITY COLLEGE CORK NATIONAL UNIVERSITY OF IRELAND CORK

<120>   Lacticaseibacillus paracasei EM205-11 and uses thereof

<130>   P13630EP00

<160>   1

<170>   PatentIn version 3.5

<210>   1
<211>   1296
<212>   PRT
<213>   Lactobacillus casei

<400>   1

```
Met Glu Met Asp Glu Lys Lys His Tyr Lys Met Tyr Lys Ser Lys Ser
1               5                   10                  15


Val Trp Val Phe Ala Cys Leu Ser Thr Cys Leu Ile Val Ser Phe Phe
            20                  25                  30


Asn Asp Gly Gln Asn Val Ser Ala Ala Thr Ser Ala Ser Ser Thr Gln
        35                  40                  45


Ile Ser Gln Thr Asn Thr Gly Ser Gln Pro Asn Asn Glu Thr Thr Gly
        50                  55                  60


Glu Thr Ala Gln Ser Ser Val Asn Ser Thr Ala Thr Ala Ser Ser Ser
65                  70                  75                  80


Ser Val Ala Asp Leu Pro Ser Ser Ser Asp Ser Lys Ser Ser Ile Gly
                85                  90                  95


Ser Thr Ile Ser Gln Pro Ala Val Asp Lys Lys Glu Thr Ser Lys Ser
            100                 105                 110


Asp Thr Ala Asp Asn Asp Leu Ile Lys Ser Val Thr Thr Ser Asp Ser
        115                 120                 125


Asp Lys Ala Leu Pro Thr Ser Lys Thr Thr Leu Pro Thr Ser Asn Glu
        130                 135                 140


Gln Val Gln Ser Ser Val Gly Gln Ser Gln Thr Asp Gln Pro Ala Ser
145                 150                 155                 160


Ser Ala Thr Ile Ala Thr Asn Ala Val Thr Ser Asp Val Ser Gln Asn
                165                 170                 175
```

Glu Gln Tyr Asn Glu Pro Tyr Arg Asn Gln Tyr His Tyr Ser Ser Ser
                180                 185                 190

Gln Asn Trp Ile Asn Asp Pro Asn Gly Leu Phe Tyr Asp Ser Lys Thr
            195                 200                 205

Gly Leu Tyr Asn Leu Tyr Tyr Gln Tyr Asn Pro Glu Gly Asn Gln Trp
        210                 215                 220

Gly Asn Met Ser Trp Gly His Ala Val Ser Lys Asp Leu Ile Asn Trp
225                 230                 235                 240

Thr Gln Glu Asp Val Ala Ile Pro Met Leu Gln Asn Gln Gly Trp Glu
                245                 250                 255

Asp Phe Thr Tyr Thr Asn Thr Thr Gly Ser Leu Lys Asp Lys Gly Glu
            260                 265                 270

Val Arg Tyr Val Gly Val Pro Thr Thr Asn Trp Gly Asp Ala Asp Gly
        275                 280                 285

Lys Lys Ala Ile Phe Ser Gly Ser Ile Val Val Asp Thr Asn Asn Val
    290                 295                 300

Ser Gly Leu Gly Lys Asp Ala Ile Leu Ala Phe Tyr Thr Ala Asp Tyr
305                 310                 315                 320

Gln Ile Ala Thr Arg Lys Asn Asp Gly Ala Glu Asp Gly Trp Gly Thr
                325                 330                 335

Trp Ile Gly Leu Thr Glu Ile Gln Glu Gln His Leu Ala Tyr Ser Leu
            340                 345                 350

Asp Gly Gly Lys Thr Phe Ile Gln Tyr Ser Lys Asp Gly Asn Ala Ala
            355                 360                 365

Asn Pro Gln Ala Ile Ile Pro Thr Ser Met Asn Gln Gly Gly Asp Ala
    370                 375                 380

Ala Asn Phe Arg Asp Pro Ser Val Val Tyr Asp Ala Val Asn Lys Gln
385                 390                 395                 400

Tyr Tyr Leu Thr Val Val Ser Gly Gln Gln Ala Leu Ile Tyr Lys Ser
            405                 410                 415

Ser Asn Leu Leu Asp Trp Thr Tyr Ala Ser Lys Ile Glu Arg Glu Asn
            420                 425                 430

Asp Val Gly Asn Gly Val Trp Glu Cys Pro Ser Leu Val Pro Met Lys
        435               440               445

Val Ala Gly Thr Asn Glu Thr Lys Trp Val Phe Cys Ile Ser Val Gln
        450               455               460

Gln Gly Ala His Ala Thr Gly Ser Gly Met Gln Tyr Tyr Val Gly Asn
465               470               475               480

Met Thr Ala Asp Gly Thr Trp Val Pro Glu Ser Ser Lys Thr Leu Gln
                485               490               495

Asn Pro Met Thr Met Asp Ser Gly Glu Asp Phe Tyr Ala Gly Ile Pro
            500               505               510

Phe Ser Asn Met Pro Asp Gly Arg Thr Val Met Leu Ala Trp Gln Ser
        515               520               525

Asn Trp Ser Tyr Val Asp Glu Ala Lys Thr Ser Pro Trp Ser Gly Asn
        530               535               540

Met Thr Leu Pro Arg Glu Leu Ser Leu Lys Lys Asn Ala Asp Thr Thr
545               550               555               560

Asp Gly Tyr Leu Leu Thr Asn Thr Val Val Lys Glu Ile Ala Asn Asn
                565               570               575

Glu Glu Ala Asn Val Ile Asn Lys Ala Glu Ser Asn Phe Thr Val Ser
            580               585               590

Arg Ser Asp Glu Gln Val Gln Tyr Glu Gly Lys Gln Tyr Lys Ile Ser
        595               600               605

Ala Thr Phe Ser Trp Asp Glu Ala Asp Lys Pro Lys Ser Val Gly Phe
    610               615               620

Lys Leu Arg Val Ser Asp Asp Gln Lys Tyr Asp Met Ile Val Gly Tyr
625               630               635               640

Asp Leu Thr Thr Gly Leu Leu Tyr Val Gln Arg Leu Asn Thr Gly Glu
                645               650               655

Pro Asn Met Gly Ala Pro Arg Asp Lys Met Asn Ala Thr Val Asn Ala
            660               665               670

Asp Gly Ser Ile Thr Ile Thr Val Tyr Val Asp Glu Thr Ser Ile Glu
        675               680               685

22

Ala Phe Ala Asn Asp Gly Glu Lys Ser Ile Thr Gln Asn Phe Phe Met
690 695 700

Arg Pro Glu Asn Ile Gly Asp Gln Ala Thr Thr Gly Val Tyr Val Tyr
705 710 715 720

Ser Asn Asp Gly Thr Thr Lys Ile Ser Asp Leu Thr Ile Asn Pro Ile
725 730 735

Thr Ser Ile Trp Asn Ser Thr Gly Gln Leu Thr Glu Lys Phe Val Asp
740 745 750

Glu Asn Gly Asn Thr Ile Ala Ser Asp Lys Ile Gln Thr Gly Arg Val
755 760 765

Gly Gln Ser Tyr Thr Ser Glu Ser Ala Thr Ile Pro Gly Tyr Val Phe
770 775 780

Val Lys Glu Asn Thr Asp His Ile Asn Ser Asn Gln Leu Tyr Thr Thr
785 790 795 800

Gln Asn Gln Thr Ile Thr Tyr Thr Tyr Arg Ala Ser Gln Ala Ser Val
805 810 815

Val Thr Lys Asp Thr Thr Leu Val Ala Gly Pro Ser Ala Ala Trp Asn
820 825 830

Ala Ala Asp Asn Leu Val Gly Ala Thr Asp Ala Asp Gly Asn Ala Leu
835 840 845

Ala Val Ser Asp Leu Thr Val Asn Gly Ala Val Asp Pro Lys Thr Pro
850 855 860

Gly Thr Tyr Thr Val Thr Tyr Ser Tyr Thr Asp Ala Thr Asp Asn Lys
865 870 875 880

Ile Ser Lys Lys Ala Thr Val Thr Val Ile Ala Ser Lys Ala Asp Ile
885 890 895

Val Thr Lys Asp Thr Thr Met Val Ala Gly Pro Ser Ala Thr Trp Asn
900 905 910

Ala Val Asp Asn Phe Val Glu Ala Thr Gly Ala Asp Gly Ser Val Leu
915 920 925

Thr Leu Ser Asp Leu Thr Val Asn Gly Ala Val Asp Pro Lys Thr Pro

930                     935                     940

Gly Thr Tyr Thr Val Thr Tyr Ser Tyr Thr Asp Ala Ala Gly Asn Lys
945                     950                     955                     960

Ile Ser Lys Glu Ala Thr Val Thr Val Ile Ala Ser Lys Ala Asp Ile
              965                     970                     975

Val Thr Lys Asp Thr Thr Met Val Ala Gly Ala Ser Thr Ile Trp Asn
              980                     985                     990

Ala Ala Asp Asn Phe Val Glu Ala  Lys Asn Ala Asp Gly  Asn Ala Leu
              995                     1000                    1005

Thr Val  Ser Asp Leu Met Ile  Asn Gly Thr Val Asp  Ser Lys Thr
         1010                    1015                    1020

Pro Gly  Thr Tyr Thr Val Thr  Tyr Ser Tyr Thr Asp  Ala Ala Gly
         1025                    1030                    1035

Asn Lys  Ile Ser Lys Glu Ala  Thr Val Thr Val Ile  Ala Ser Lys
         1040                    1045                    1050

Ala Asp  Ile Val Thr Lys Asp  Thr Thr Met Val Ala  Gly Pro Ser
         1055                    1060                    1065

Ala Thr  Trp Asn Ala Ala Asp  Asn Leu Val Ile Ala  Thr Asp Ala
         1070                    1075                    1080

Lys Gly  Asn Ala Leu Ala Leu  Ser Asp Leu Thr Val  Asn Gly Ala
         1085                    1090                    1095

Val Asp  Pro Lys Thr Pro Gly  Thr Tyr Thr Val Thr  Tyr Ser Tyr
         1100                    1105                    1110

Thr Asp  Pro Ala Gly Asn Lys  Ile Ser Lys Glu Ala  Thr Val Thr
         1115                    1120                    1125

Val Ile  Ala Ser Lys Ala Asp  Ile Val Thr Lys Asp  Thr Thr Met
         1130                    1135                    1140

Val Ala  Gly Pro Ser Ala Ala  Trp Asn Ala Ala Asn  Asn Leu Val
         1145                    1150                    1155

Ser Ala  Thr Asp Ala Asp Gly  Asn Ala Leu Ala Met  Ser Asn Leu
         1160                    1165                    1170

```
Thr Val  Thr Gly Thr Val Asp  Leu Lys Thr Gln Gly  Ala Tyr Thr
    1175             1180             1185

Val Thr  Tyr Thr Tyr Thr Asp  Val Ala Gly Asn Lys  Ile Ser Lys
    1190             1195             1200

Glu Ala  Thr Val Thr Val Leu  Thr Glu Lys Glu Thr  Asn Ile Glu
    1205             1210             1215

Asp Asn  Thr Gly Ser Ser Ile  Ser Asn Asp Arg Glu  Asn Pro Pro
    1220             1225             1230

Ala Ser  Ile Thr Gly Lys Gly  Gly Asp Asp Ile His  Gln Asn Ala
    1235             1240             1245

Lys Thr  Thr Met Thr Lys Lys  Lys Thr Glu Thr Leu  Pro Gln Ala
    1250             1255             1260

Gly Asn  His Val Asn Glu Leu  Ala Ile Val Leu Gly  Gln Met Ile
    1265             1270             1275

Leu Ala  Ile Cys Val Gly Gly  Ile Leu Trp Leu Lys  Arg Arg Val
    1280             1285             1290

Lys Arg  Val
    1295
```